(19) Europäisches Patentamt
European Patent Office
Office européen des brevets

(11) **EP 4 703 000 A1**

(12) **EUROPEAN PATENT APPLICATION**
published in accordance with Art. 153(4) EPC

(43) Date of publication:
04.03.2026  Bulletin 2026/10

(21) Application number: 23934515.0

(22) Date of filing: 27.04.2023

(51) International Patent Classification (IPC):
*A61M 21/02* *(2006.01)*

(52) Cooperative Patent Classification (CPC):
(C-Sets available)
G16H 20/70; A61B 5/165; A61M 21/00;
A61M 21/02; A61N 5/0618; G06V 40/174;
G16H 40/63; G16H 50/20; H05B 45/20;
H05B 47/105; H05B 47/11; H05B 47/115;
H05B 47/155; H05B 47/175; H05B 47/19;    (Cont.)

(86) International application number:
PCT/CN2023/091059

(87) International publication number:
WO 2024/221314 (31.10.2024 Gazette 2024/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(60) Divisional application:
25169032.7

(71) Applicant: Lin, Lawrence
Taoyuan City, Taiwan 330010 (CN)

(72) Inventors:
• CHANG, Chih Hung
  New Taipei City Taiwan (TW)
• CHANG, Kun-Chieh
  New Taipei City Taiwan (TW)
• LIN, Lawrence
  New Taipei City Taiwan (TW)

(74) Representative: Lang, Christian
LangPatent Anwaltskanzlei IP Law Firm
Ingolstädter Straße 5
80807 München (DE)

(54) **EMOTION NAVIGATION SYSTEM USING INTELLIGENT HUMAN CENTRIC LIGHTING, AND METHOD THEREFOR**

(57)    The present invention discloses a method for implementing emotion navigation by using an intelligent human centric lighting system. The emotion navigation method includes the following steps. Step 1 is to confirm an initial emotion of a user. Step 2 is to set a target emotion. Step 3 is to select a relay emotion to set an emotion navigation path. Step 4 is to edit a multispectral recipe corresponding to the relay emotion and the target emotion according to the multispectral recipe. Step 5 is to perform a lighting program of the multispectral recipe of the relay emotion and the target emotion. If the determination is not reaching the target emotion of Step 6, then go to obtain the user's personal physiological data to find out the multispectral recipe of the corresponding emotion that can adjust to the target emotion.

FIG.5

(52) Cooperative Patent Classification (CPC): (Cont.)
A61M 2021/0044; A61M 2205/3553;
A61M 2205/3592; A61M 2205/505; A61M 2205/52;
A61M 2205/587; A61M 2209/088; A61M 2230/10;
A61M 2230/63; A61N 2005/0626;
A61N 2005/0636; G06F 2218/00

C-Sets
A61M 2230/10, A61M 2230/005;
A61M 2230/63, A61M 2230/005

**Description**

FIELD OF THE INVENTION

The present invention relates to an intelligent human centric lighting method, particularly relates to an intelligent human centric lighting method that measures the emotion state of human physiological signals so as to adjust the lighting spectrum, and then achieves emotion navigation that transfers emotions through the intelligent human centric lighting process.

BACKGROUND OF THE INVENTION

Humans are animals with changeable emotions, they will have different emotional reactions with the individual's psychological state, such as excitement, amusement, anger, disgust, fear, happiness, sadness, serene, neutral. When negative emotions (such as anger, disgust and fear) cannot be resolved in time, they will cause psychological damage or trauma to the human body, and finally evolve into mental illness. Therefore, how to timely provide an emotional resolution or relief or treatment system that can meet the needs of users has broad business opportunities in today's society, full of high competition and high pressure at any time.

In modern medical equipment, the changes of hemodynamics caused by neuronal activity can be measured by functional Magnetic Resonance Imaging (fMRI) system. Due to the non-invasiveness of fMRI and its low radiation exposure, fMRI is currently mainly used in the study of human and animal brain or spinal cord. At the same time, the tester can also be checked by the electroencephalogram of the EEG, and the emotions can be stimulated in the same way, and the responses of different emotions can be seen, for example, the brainwave patterns of fear and happiness can be seen significantly different. Among them, when observing the response of a certain emotion under fMRI and EEG, for example, under happy emotion (which can be induced by pictures and matched with facial emotion recognition), fMRI was used to observe the blood oxygen-level dependent (BOLD) contrast response and found that in the medial prefrontal cortex (Mpfc), there were significantly more responses to the corresponding emotions (anger and fear). In contrast, for example, in the case of fear and anger, fMRI was used to observe the blood oxygen-level dependent (BOLD) contrast response, and it was found that it was significantly reflected in the amygdala region, showing that the two kinds of emotions have different response regions in the brain. Therefore, the blood oxygen-level dependent (BOLD) contrast response of different regions in the brain can be used to clearly determine which emotion the tester is currently in. In addition, if the electroencephalogram of the EEG is used to examine the measurement tester, and the emotions are stimulated in the same way, it can be seen that the brainwave patterns of fear and happiness are significantly different. Therefore, you can also determine what kind of emotion the tester is currently in through the brainwave patterns of different reactions. According to the above, for the functional Magnetic Resonance Imaging (fMRI) system, emotions are distinguished by different blood oxygen-level dependent (BOLD) contrast reactions, while for the EEG, emotions are distinguished by different brainwave patterns. It is obvious that the methods used to determine the emotion of the tester and the contents recorded are completely different. Therefore, in terms of current science and technology, the brainwave patterns of EEG cannot replace the blood oxygen-level dependent (BOLD) contrast response of functional Magnetic Resonance Imaging (fMRI) for the test results of the same emotion of the same tester.

The above discussion on emotion determination by functional Magnetic Resonance Imaging (fMRI) system and EEG is that fMRI system is very expensive and huge, so it cannot be used in commercial systems and methods of human centric lighting. Similarly, if only the brainwave patterns of the EEG are used to determine the mood of the tester, it may be encountered that the brainwave patterns of different testers for different emotions may be different. Therefore, at present, it is impossible to use the blood oxygen-level dependent (BOLD) contrast response of functional magnetic resonance imaging system (fMRI) alone, or the brain wave mode of EEG alone to construct a commercial human centric lighting method and system through the editing of light recipe.

SUMMARY OF THE INVENTION

According to the above description, the present invention provides a method to establish a correlation between the brainwave pattern of an EEGthe blood oxygen-level dependent (BOLD) contrast of a functional Magnetic Resonance Imaging (fMRI), after which the brainwave pattern of an EEG is used as a human centric lighting application on a commercial system. Next, the present invention further provides an emotional navigation method and system that can transfer the user's emotions through the lighting process of intelligent human centric.

An object of the present invention is to provide a method for implementing emotion navigation by using an intelligent human centric lighting system. The emotion navigation method includes the following steps. Step 1 is to confirm an initial emotion of a user. Step 2 is to set a target emotion. Step 3 is to choose a relay emotion to set the emotion navigation path. Step 4 is to edit a multispectral recipe corresponding to the relay emotion and the target emotion according to the emotion navigation path. Step 5 is to perform a lighting program of the multispectral recipe. Step 6 is to determine if the user's emotion reaching the target emotion. If the determination is not reaching the target emotion of Step 6, then go to obtain the user's personal physiological data to find out the multispectral recipe of the corresponding emotion that can adjust to the target emotion. Then go to re-execute Step 4 to Step 6 until the user's emotion reaching the target emotion, then stop the lighting program.

One object of the present invention is to provide a method for implementing emotion navigation by using an intelligent human centric lighting system. The intelligent human centric lighting system is composed of a cloud, a lighting field end and a client terminal which are connected to each other through the Internet. An emotion coordinate information is stored in the cloud. The emotion navigation method includes the following steps. Step 1 is to confirm an initial emotion of a user through a physiological monitoring device or by using an International Affection Picture System (IAPS) to simulate for confirming the initial emotion of the user. The initial emotion stores in the cloud. Step 2 is to set a target emotion according to a requirement of the user by the client terminal. The target emotion stores in the cloud. Step 3 is to choose a relay emotion to set an emotion navigation path. The relay emotion stores in the cloud. Wherein, the client terminal is connected the cloud through the Internet and the relay emotion is chosen from an emotion coordinate information in the cloud. Step 4 is to edit a multispectral recipe according to the light recipe of the relay emotion and the light recipe of the target emotion to edit the multispectral recipe corresponding to the relay emotion and the multispectral recipe corresponding to the target emotion. Step 5 is to perform a lighting program of the multispectral recipe corresponding to the relay emotion. Wherein, the client terminal controls a lamps group in the lighting field end to conducting a lighting process to the user for a set time according to the multispectral recipe corresponding to the relay emotion. Step 6 is to determine if the user's emotion reaching a neutral emotion through the physiological monitoring device. Step 7 is to perform a lighting program of the multispectral recipe corresponding to the target emotion. After the determination of the user's emotion is reaching the neutral emotion, the client terminal controls a lamps group in the lighting field end to conduct a lighting process to the user for a set time according to the multispectral recipe corresponding to the target emotion. Step 8 is to determine if the user's emotion reaching the target emotion through the physiological monitoring device. Step 9 is to stop lighting when determination of the user's emotion is reaching the target emotion.

One object of the present invention is to provide a method for implementing emotion navigation by using an intelligent human centric lighting system. The intelligent human centric lighting system is composed of a cloud, a lighting field end and a client terminal which are connected to each other through the Internet. An emotion coordinate information and users' personal biological data are stored in the cloud. The emotion navigation method includes the following steps. Step 1 is to confirm an initial emotion of a user through a physiological monitoring device or by using an International Affection Picture System (IAPS) to simulate for confirming the initial emotion of the user. The initial emotion stores in the cloud. Step 2 is to set a target emotion according to a requirement of the user by the client terminal. The target emotion stores in the cloud. Step 3 is to obtain the user's personal biological data stored in a memory module of the cloud by the client terminal. Step 4 is to choose a relay emotion to set an emotion navigation path and storing the relay emotion in the cloud. Wherein, the client terminal is connected the cloud through the Internet and the relay emotion is chosen from an emotion coordinate information in the cloud. Step 5 is to edit a multispectral recipe according to the light recipe of the relay emotion and the light recipe of the target emotion and the user's personal biological data to edit the multispectral recipe corresponding to the relay emotion and the multispectral recipe corresponding to the target emotion. Step 6 is to perform a lighting program of the multispectral recipe corresponding to the relay emotion. Wherein, the client terminal control a lamps group in the lighting field end to conducting a lighting process to the user for a set time according to the multispectral recipe corresponding to the relay emotion. Step 7 is to determine if the user's emotion reaching a neutral emotion through the physiological monitoring device. Step 8 is to perform a lighting program of the multispectral recipe corresponding to the target emotion. Wherein, the client terminal control a lamps group in the lighting field end to conducting a lighting process to the user for a set time according to the multispectral recipe corresponding to the target emotion. Step 9 is to determine if the user's emotion reaching the target emotion through the physiological monitoring device. Step 10 is to stop lighting when determination of the user's emotion is reaching the target emotion.

In the above methods of implementing emotion navigation, it can further use the personal physiological data for adjusting the lighting parameters of the multispectral recipe.

In the above methods of implementing emotion navigation, the multispectral recipe includes lighting parameters such as color temperature, illuminance(I), flicker frequency(H), and color rendering (Ra).

In the above methods of implementing emotion navigation, the multispectral recipe is a formula formed by the surrounding system score (LSS).

$$LSS = t \times [a \times f_{eeg}(freq) + b \times f_{eeg}(Ra) + c \times f_{eeg}(CTI) + d \times f_{eeg}(I)]$$

$a = w_{freq}/w_{CTI}$
$b = w_{Ra}/w_{CTI}$

$$c = W_{CTI}/W_{CTI} = 1$$
$$d = w_I/w_{CTI}$$
$$t = \text{illumination time}$$

The method of emotion navigation of the present invention is that after determining the current emotion of the user, in the first stage, the relay emotion spectrum irradiation with complementary emotions to the initial emotion is given. Then proceed to the second stage of spectral irradiation of the target emotion for the user to reach the target emotion. In particular, when it is further confirmed that the user has already shifted his emotion to a neutral emotion after being irradiated with the spectrum of complementary emotions in the first stage. Afterwards, the second stage of spectral irradiation of the target emotions can be carried out, which can greatly improve the indicators of the target emotions. In addition, when the neutral emotion has not been reached after the irradiation of the relay emotion, the user's personal physiological data can be further provided for adjusting the lighting parameters of the color temperature, illuminance, flicker frequency, and color rendering (Ra). The user's personal physiological data is a kind of physiological data of the user's health check, after confirming that the emotion has been transferred to the neutral emotion or near the origin of the emotion coordinates system, the index of the target emotion can be greatly improved.

BRIEF DESCRIPTION OF THE DRAWINGS

FIG.1a is the original data collection structure of the human centric lighting to physiological and emotional according to the present invention.
FIG.1b is the original data collection of the human centric lighting to physiological and emotional according to the present invention.
FIG.1c is the determination flow for the response of human centric lighting to specific physiological and emotional according to the present invention.
FIG.2a is a method for establishing the response of EEG of human centric lighting to physiological and emotional.
FIG.2b is the lighting database that the present invention constructs the user to carry out effective human centric lighting.
FIG.3 is the system frame diagram of the intelligent human centric lighting system of the present invention.
FIG.4a is the emotion coordinate system of the present invention.
FIG.4b is the electroencephalogram of the specific emotion of the present invention.
FIG.4c is the electroencephalogram of emotion transfer test 1 of the present invention.
FIG.4d is the electroencephalogram of emotion transfer test 2 of the present invention.
FIG.4e the electroencephalogram of emotion transfer test 3 of the present invention.
FIG.4f is the navigation path of the emotional navigation example 1 of the present invention.
FIG.4g is the navigation path of the emotional navigation example 2 of the present invention.
FIG.4h is the navigation path of the emotional navigation example 3 of the present invention.
FIG.5 is a method for performing emotional navigation according to the present invention.
FIG.6 is a method for performing emotional navigation according to the present invention; and
FIG.7 a method for performing emotional navigation according to the present invention.

DETAILED DESCRIPTION OF THE PREFERRED EMBODIMENT

In the specification after the present invention, the functional magnetic resonance imaging system is referred to as "fMRI system", the Electroencephalography is referred to as "EEG", and the blood oxygen-level dependent comparison is referred to as "BOLD". In addition, in the embodiment of the color temperature test of the present invention, the test is carried out in units of every 100K. However, in order to avoid too lengthy description, in the following description, the so-called specific emotion refers to excitement or excitement, happiness, amusement, etc., and the corresponding specific emotional response will be explained with 3,000K, 4,000K and 5,700K as color temperature test examples, therefore, the present invention cannot be limited to the embodiments of the three color temperatures. At the same time, in order to make those in the technical field of the present invention fully understand the technical content, relevant embodiments and embodiments thereof are provided for explanation. In addition, when reading the embodiment provided by the present invention, please also refer to the schema and the following description, in which the shape and relative size of each component in the schema are only used to assist in understanding the content of the embodiment, not to limit the shape and relative size of each component.
The present invention uses fMRI system to understand the corresponding relationship between spectrum and emotion in the brain through physiological signal measurement method, and formulates a set of preliminary mechanism of using light to affect human physiological and psychological reactions, because the brain imaging of fMRI system can determine which part of the human brain has hyperemia reaction when stimulated by light, it can also record BOLD reaction. This BOLD response to brain hyperemia is also known as the "rise of blood oxygen concentration dependent response". Therefore, according to the image recording data of brain hyperemia under various emotions of fMRI system and the response of "rise of blood oxygen-level dependent response", the present invention can accurately and objectively infer the physiological and emotional changes of the tester, and then take the physiological and emotional confirmed by fMRI system as the basis, further, EEG is used to record the changes of EEG to establish the correlation between them, in order to use the changes of EEG to replace the emotion determination of fMRI system.

Therefore, the main purpose of the present invention is to enable the tester to record the BOLD response of the tester to the specific emotion after illuminating the tester during the test of specific emotion in the fMRI system, so as to screen out which specific "effective color temperature" can multiply the specific emotion, the color temperature of lighting is used as the "effective color temperature" corresponding to specific emotions. After that, the tester is illuminated with "effective color temperature", and the EEG is used to record the brainwave patterns under the stimulation of "effective color temperature", so that the specific brainwave patterns of the EEG is related to the specific BOLD response. After that, the user's emotional change can be assisted by the specific EEG mode of the EEG, in order to construct a set of intelligent human centric lighting system and its method that can be operated commercially, so as to solve the problem that expensive fMRI system must be used to execute intelligent human centric lighting system, which can reduce the operation cost and further meet the customized service demand.

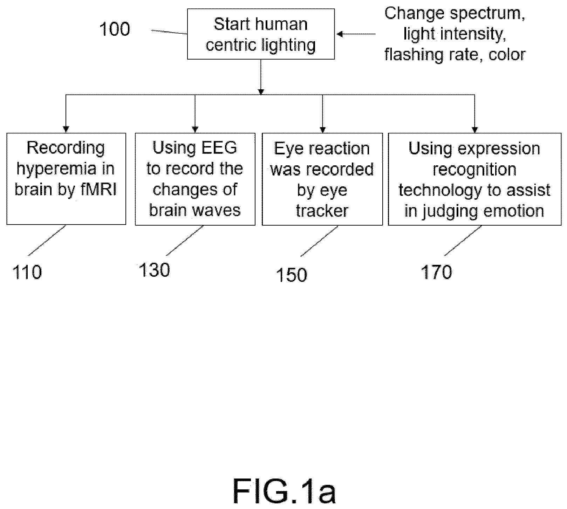

First, please refer to FIG.1a, which is the original data collection framework of physiological and emotional due to human centric lighting of the present invention. As shown in FIG.1a, starting the intelligent human centric lighting system 100 is in an environment where various adjustable lighting modules have been configured (e.g., a test space), and provides light signal lighting parameters such as spectrum, light intensity, flicker rate and color temperature that can be changed. For different target emotions, the present invention uses the fMRI compatible image interaction platform 110 formed by the fMRI system to guide the emotion of voice and image, and at the same time, it is matched with a specific effective spectrum to stimulate for 40 seconds. Observe the changes in the area where the blood oxygen-level changes in the tester's brain to verify whether the "effective color temperature" can significantly induce the tester's emotional response, and the details are as follows.

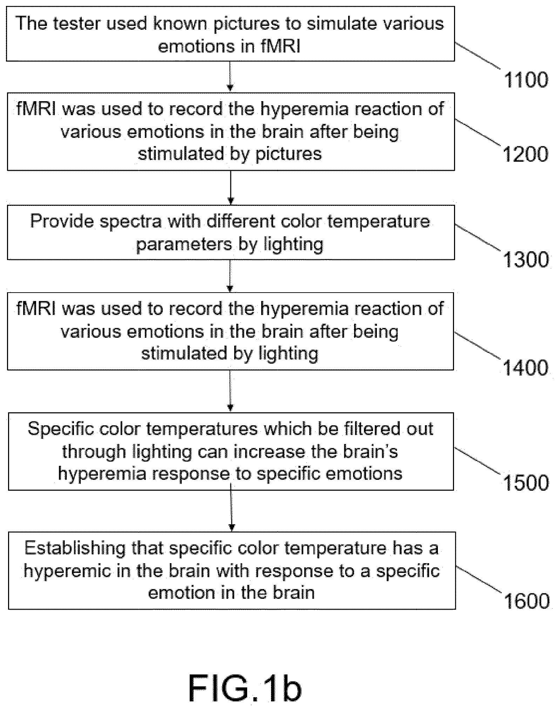

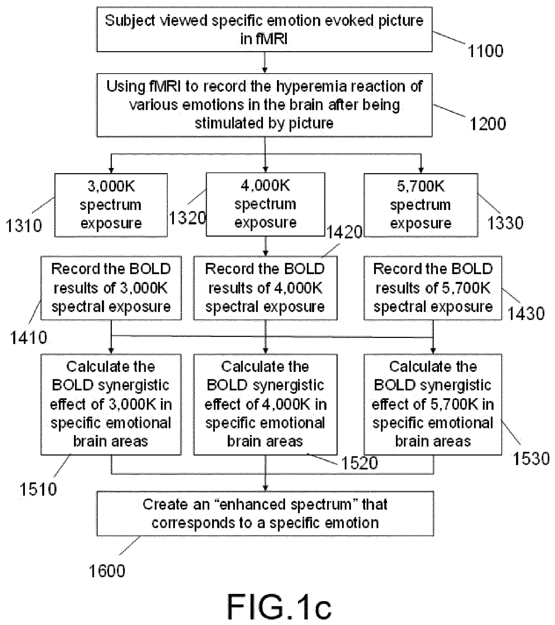

Next, please refer to FIG.1b and FIG.1c, wherein FIG.1b is a flow chart of collecting original data of human centric lighting to physiological and emotional responses according to the present invention, and FIG.1c is a determining flow of human centric lighting to specific physiological and emotional responses. As shown in step 1100 in FIG.1b, each tester has been positioned on the compatible image interaction platform 110, and then each tester is guided to stimulate various emotions through known pictures. Afterwards, as shown in step 1200, the BOLD response in the brain of the tester's various emotions after being stimulated by the picture is recorded through the compatible image interaction platform 110. Next, as shown in step 1300, the tester is visually stimulated by irradiating light to provide spectrums with different color temperature parameters. For example: use LED lamps with electronic dimmers to provide spectrums with different color temperature parameters. In the embodiment of the present invention, nine groups of visual stimuli with different color temperatures, including 2,700K, 3,000K, 3,500K, 4,000K, 4,500K, 5,000K, 5,500K, 6,000K, and 6,500K, are provided. Among them, after completing 40 seconds of effective light irradiation and stimulation each time, you can choose to give testers one minute of invalid light source lighting (full spectrum non-flicker white light) to achieve the purpose of emotional relaxation. Further, it is also possible to choose to give the tester a 40 second counter effect light stimulation to observe whether the response in the area that responded to the original effective light stimulation decreased. In the embodiment of the present invention, after the compatible image interaction platform 110 has recorded the BOLD response in the brain of the tester's specific emotion after being stimulated by the picture, the tester is visually inspected by providing spectra with different color temperature parameters. The stimulus, as shown in step 1310 in FIG.1c, is used to provide a spectrum with a color temperature of 3,000K, then, as shown in step 1320, to provide a spectrum with a color temperature of 4,000K, and finally, as shown in step 1330, to provide a color temperature for the 5,700K spectrum, the test subjects were visually stimulated.

Next, as shown in step 1400, the BOLD response of the test subject's brain after being stimulated by light is recorded through the compatible image interaction platform 110. In the embodiment of the present invention, after the tester is stimulated by lighting with different color temperature parameters, the compatible image interaction platform 110 records the BOLD response results with emotional response areas in the corresponding limbic system of the tester in sequence. Among them, the location of limbic system triggered by different emotions is different, and the above limbic system with emotional response in brain area is shown in Table 1 below.

Table 1

| Limbic system | Function |
|---|---|
| ACC | Emotion |

(continued)

| Limbic system | Function |
|---|---|
| Cuneus | Vision, Control bipolar disorder |
| Cerebellum | Balance, Coordination, Antidepressant & Anxiety |
| Insula Lobe | Affection, addiction |
| Lingual Gyrus | Logic, Vision ' Antidepressant & Anxiety |
| Rolandic Operculum | Emotion Management, Gustatory Sense |
| Superior Frontal Gyrus | Working Memory, Self-consciousness, laugh |
| Middle Temporal Gyrus | Emotion Recognition, Creativity |
| MCC | Emotion |
| Pallidum | Affection, Motion, Cognition, Reward System |
| Temporal Pole | Affection, Language, Auditory |

The compatible image interaction platform 110 records the response result of BOLD in a specific area of the brain, and the response result is determined by calculating the area of these emotional response parts when the brain area has more limbic system with BOLD emotional response, for example: when there is a BOLD emotional response The larger the area of emotional response, the stronger the response to a specific physiological and emotional. In the embodiment of the present invention, as shown in step 1410 in FIG.1c, it is used to record the reaction result of BOLD with a color temperature of 3,000K after spectral irradiation, and then, as shown in step 1420, it is used to record the color temperature of 4,000K. Finally, as shown in step 1430, the reaction result of BOLD after spectral irradiation is used to record the reaction result of BOLD after spectral irradiation with a color temperature of 5,700K. Among them, after the tester passes through the lighting procedure after the excitement is induced, the compatible image interaction platform 110 records the response results of BOLD in a specific area of the brain as shown in Table 2. Obviously, when the color temperature of 3000K is irradiated, the excitement can be enhanced. Therefore, according to the results of the embodiments of the present invention, the optimal stimulating color temperature is between 3000K and 4000K. However, it should be noted that after exposure to a color temperature of 5700K, there will be a negative inhibitory effect on the excitement.

Table 2

| Excitement Index | | | |
|---|---|---|---|
| Limbic system | 3,000K | 4,000K | 5,700K |
| ACC | 0 | 0 | -30 |
| Cerebellum | 577 | 0 | -75 |
| Middle Temporal Gyrus | 0 | 99 | 0 |
| Superior Frontal Gyrus | 0 | 127 | 0 |
| Total T Score | 577 | 226 | -105 |

Among them, after the tester passes through the lighting procedure induced by happiness, the compatible image interaction platform 110 records the response results of BOLD in a specific area of the brain as shown in Table 3. Obviously, when the color temperature of 4000K is irradiated compared with the other color temperatures, the color temperature of 4000K can enhance the BOLD response of the brain area in the happiness. Therefore, according to the results of the embodiments of the present invention, the optimal stimulating color temperature of happiness is around 4000K.

Table 3

| Happiness Index | | | |
|---|---|---|---|
| Limbic system | 3,000K | 4,000K | 5,700K |
| ACC | 0 | 0 | -162 |
| Cerebellum | 163 | 252 | 0 |

(continued)

| Happiness Index | | | |
|---|---|---|---|
| Limbic system | 3,000K | 4,000K | 5,700K |
| Pallidum | 80 | 0 | 0 |
| Insula Lobe | 85 | 307 | 0 |
| Lingual Gyrus | 0 | 58 | 403 |
| Temporal Pole | 0 | 57 | 0 |
| Total T Score | 328 | 674 | 241 |

Among them, after the tester passes through the lighting procedure after the emotion-induced amusement, the compatible image interaction platform 110 records the response results of BOLD in a specific area of the brain as shown in Table 4. Obviously, all color temperatures can enhance the BOLD response of the brain area of amusement, especially when the color temperature is higher, the BOLD response of the brain area of amusement is stronger.

Table 4

| Amusement Index | | | |
|---|---|---|---|
| Limbic system of brain | 3,000K | 4,000K | 5,700K |
| Lingual Gyrus | 0 | 279 | 327 |
| Cerebellum | 539 | 215 | 226 |
| Middle Temporal Gyrus | 0 | 0 | 90 |
| MCC | -83 | 32 | 0 |
| Total T Score | 456 | 526 | 643 |

Among them, after the tester passed the lighting program induced by the emotion of the serene index, the compatible image interaction platform 110 recorded the response results of BOLD in specific areas of the brain, as shown in Table 5. Obviously, when the low color temperature of 3000K is irradiated, it can enhance the sense of tranquility or relaxation. However, when the color temperature is higher, it will have a negative inhibitory effect on the mood of the serene. In particular, the higher the color temperature, the more negative the suppression effect will be.

Table 5

| Serene Index | | | |
|---|---|---|---|
| Limbic system of brain | 3,000K | 4,000K | 5,700K |
| ACC | 0 | 32 | 0 |
| Cerebellum | 151 | 0 | 221 |
| Cuneus | 0 | 0 | -789 |
| Lingual Gyrus | 298 | 0 | 107 |
| Middle Temporal Gyrus | 0 | -134 | 0 |
| Superior Frontal Gyrus | 0 | 0 | -598 |
| Total T Score | 449 | -102 | -1059 |

Afterwards, as shown in step 1500, the results of the BOLD reaction that a specific color temperature can increase a specific emotion are screened by lighting, and the specific color temperature is called an "effective color temperature". In this embodiment, the BOLD response result of the emotional response area in the limbic system of the tester's corresponding brain is recorded, so as to summarize the stimulation effect of color temperature on the brain, as shown in Tables 2 to 5 shown. For the BOLD brain region-dependent response results that screened out the specific color temperature that can increase a specific emotion, we calculated those specific color temperature that can make the response effect of a specific emotion reach the maximum response value (that is, the maximum response area value with BOLD).

As shown in step 1510, when the tester has recorded the excitation emotion on the compatible image interaction platform 110 and finishes the lighting program again, the maximum response value is calculated. For example, according to the records in Table 2, the total score of 3,000K (577) is subtracted from the total score of 4,000K (226) to obtain 351. Then, after subtracting the total score (- 105) of 5,700K from the total score (577) of 3,000K, 682 is obtained. Therefore, the total score of the response value after the excitement is induced under the illumination of 3000K is 1033.

Next, as shown in step 1520 in FIG.1c, when the tester has recorded the excitation emotion on the compatible image interaction platform 110 and completed the lighting program again, it starts to calculate the maximum response value. For example, according to the records in Table 2, subtract the total score (226) of 4,000K from the total score (577) of 3,000K to obtain - 351. Then, after subtracting the total score (- 105) of 5700K from the total score (266) of 4,000K, 371 is obtained. Therefore, the total score of the response value after the excitement is induced under the illumination of 4000K is 20.

Then, as shown in step 1530 in FIG.1c, after the tester has recorded the excitation emotion induction on the compatible image interaction platform 110 and completed the lighting program, it starts to calculate the maximum response value. For example, according to the records in Table 2, after subtracting the total score (- 105) of 5,700K from the total score (577) of 3,000K, it obtains - 682. Then, the total score (- 105) of 5,700K is subtracted from the total score (266) of 4,000K to obtain -371. Therefore, the total score of the response value after the excitement emotion is induced under the illumination of 5700K is -1033.

According to the above calculation, after the excitation emotion is induced, the excitation emotion can reach the maximum response value at 3,000K lighting. That is, 3,000K illuminance can make excitement get a more obvious additive effect (that is, compared with the total calculated score of 4,000K and 5,700K illuminance, the total calculated score of 3,000K illuminance is 1033, the highest). Therefore, 3,000K illuminance is used as the "effective color temperature" of excitement. For other emotions, such as "effective color temperature" of happiness, amusement and serene, different "effective color temperatures" can be obtained from the above calculation results in steps 1510 to 1530, as shown in Table 6 below.

Table 6

| Emotion | Effective Color Temperature |
|---|---|
| Excitement | 3,000K |
| Happiness | 4,000K |
| Amusement | 5,700K |
| Serene | 3,000K |

Next, according to the statistical results in Table 6, the effective color temperature can be regarded as the result of a specific physiological and emotional-dependent response, and this effective color temperature can be regarded as the "enhanced spectrum" of the "blood oxygen-level dependence" of fMRI on a certain emotion. Wherein, the optimal stimulating color temperature should fall between 3000K and 4000K. For example, an effective color temperature of 3,000K can represent the "enhanced spectrum" of the fMRI system in "excited" emotions. For example, an effective color temperature of 4,000K can represent the "enhanced spectrum" of the fMRI system in "happy" emotions. Wherein, the optimal stimulating color temperature of happiness should fall around 4000K. For example, the effective color temperature of 5,700K can represent the "enhanced spectrum" of the fMRI system in "amusement" emotions. For example, an effective color temperature of 3,000K can represent the "enhanced spectrum" of the fMRI system in "serene" emotions. Wherein, the optimal stimulating color temperature of serene should fall around 3000K.

Finally, as shown in step 1600, the light recipe database of the enhanced spectrum corresponding to the effect of a particular emotion can be established in the fMRI system. The tester is stimulated by the above-mentioned human centric lighting parameters, and the BOLD response of the tester's brain when the tester's brain is stimulated by light is observed and recorded through the fMRI system. The additive and multiplicative response of "blood oxygen-level dependence increase" makes a specific effective color temperature can be regarded as the "enhanced spectrum" of the "light recipe database" of fMRI for a specific emotion. Obviously, the present invention objectively deduces the tester's "light recipe" under a specific physiological and emotional response based on the statistical result of the BOLD reaching a specific emotional response at a specific "effective color temperature" in Table 6, and This "light recipe" is used as the evidence of the most synergistic physiological and emotional response to a specific emotion. (Including: excitement, happiness, amusement, anger, disgust, fear, sadness, calm, or neutrality)

It should be emphasized that, in the entire implementation process of FIG.1b and FIG.1c, the statistical results in Table 5 are obtained after 100 testers are respectively subjected to a complete test of multiple specific emotions. For example, in terms of excitement, providing an effective color temperature of 3,000K as the "enhanced spectrum" under the excited physiological as the light recipe and emotional response can make the tester's excited emotions produce the most synergistic physiological-emotional response. For example, in terms of happy emotions, providing an effective color temperature of 4,000K as the "enhanced spectrum" under the happy physiological as the light recipe and emotional response can make the tester's happy emotions produce physiological and emotional responses with the strongest synergistic effect. Another example: in terms of amusement emotions, providing an effective color temperature of 5,700K as an "enhanced spectrum" under the amusement physiological as the light recipe and emotional response can make the tester's amusement emotions produce a physiological and emotional response with the strongest synergistic effect.

In addition, it should also be emphasized that the above-mentioned three color temperatures are only representative of the embodiments of the present invention, and not only the lighting of the three color temperatures are used as the "enhanced spectrum" under the three physiological and emotional responses. In fact, the whole process of FIG. 1 and FIG.1c can be carried out for different emotions (including excitement, happiness, amusement, anger, disgust, fear, sadness, calm or neutral) after 2,000K color temperature is increased by 100K as an interval. Therefore, Table 5 of the present invention is only the result of the disclosure part, not to limit the present invention. The invention is only limited to these embodiments.

Next, the present invention is to establish an artificial intelligence model of "the correlation between brainwaves and brain images of general physiological and emotional", so that in future commercial promotion, the results of other sensing devices can be directly used to infer physiological and emotional without using fMRI system. Among them, the sensing device matched with the present invention includes electroencephalography (EEG). In the following embodiments, the electroencephalography (EEG) 130 is used to establish the human centric physiological and emotional response to light, and the eye tracker 150 or the expression recognition technology auxiliary program 170 can be used to replace the fMRI system's response to physiological and emotional. However, the eye tracker 150 or the expression recognition technology auxiliary program 170 will not be disclosed in the present invention, but will be announced first.

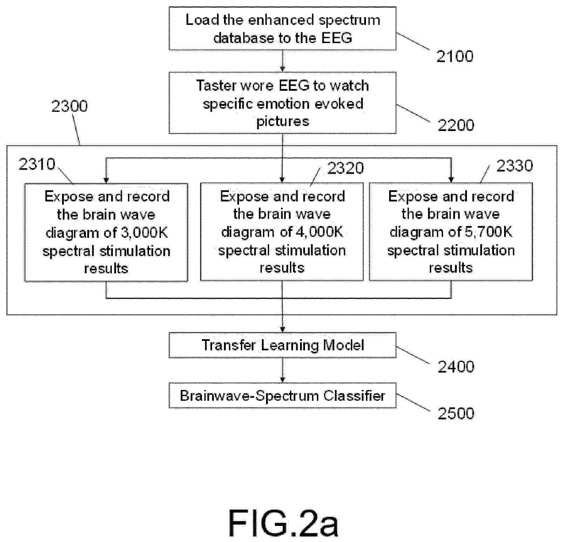

Please refer to FIG.2a, which is a method for establishing a human centric lighting electroencephalogram response to physiological and emotional according to the present invention. As shown in FIG.2a, the present invention is a method for establishing the human centric lighting response to physiological and emotional through the EEG 130, including: first, as shown in step 2100, the "enhanced spectrum" database information in Table 5 is also stored in the memory of the EEG 130. Next, as shown in step 2200, let the testers wear the EEG, and guide each tester to stimulate various emotions through the elements of known pictures or videos. For example, the emotional stimuli of known pictures or videos can be selected from the International Affection Picture System (IAPS). Afterwards, as shown in step 2300, the intelligent human centric lighting system 100 is activated, and light signal parameters such as spectrum, light intensity, flicker rate, and color temperature that can be changed are provided to stimulate the tester with light. Next, the electroencephalogram file after being illuminated with different color temperatures under a specific emotion is recorded by the EEG 130. For example, in this embodiment, each tester is first stimulated with excitement, and then, in steps 2310 to 2330, different color temperatures of 3,000K, 4,000K and 5,700K are provided to stimulate the tester respectively, and the tester is recorded in when stimulated by excitement, the electroencephalogram file of the specific emotion after the tester is illuminated with different color temperatures is stored in the device in the memory of the EEG 130. In the embodiment of the present invention, the electroencephalogram files after 100 testers have completed specific emotional stimulation and lighting have been recorded respectively, therefore, a larger memory is required.

Next, as shown in step 2400, the electroencephalogram files of specific emotions (e.g., excitement, happiness, amusement) stored in the memory of the EEG 130 is learned through the learning method of artificial intelligence. Since the EEG 130 can only store the waveform of the electroencephalogram, the electroencephalogram currently stored in the memory of the EEG 130 is the electroencephalogram file after a known specific triggering emotional stimulus and lighting of different color temperatures. It should be noted that, in the actual test, different testers have different electroencephalogram files for the same emotional stimulus and lighting with the same color temperature. Therefore, during the learning process of step 2400, the present invention needs to classify the electroencephalogram files of specific emotions through the information of the light recipe database the "enhanced spectrum". Group the electroencephalogram files with a color temperature of 3,000K, for example: For the electroencephalogram files of happy emotions, only group the electroencephalogram files of different testers with a color temperature of 4,000K. Another example: for the electroencephalogram file of happy mood, only the electroencephalogram files of different testers at 4,000K color temperature are grouped; for example, for the electroencephalogram file of happy mood, only the electroencephalogram files of different testers at 5,700K color temperature are grouped. Then, the EEG is trained through machine learning in artificial intelligence. In the embodiment of the present invention, in particular, a transfer learning model is selected for learning and training.

In the process of learning and training using the transfer learning model in step 2400, the group of electroencephalogram files for specific emotions is learned and trained by counting, calculating and comparing the similarity. For example, when learning and training the group of electroencephalogram files with 3,000K color temperature, it is based on statistics, calculation and comparison of the ranking of the highest similarity and the lowest similarity among the electroencephalogram files with 3,000K color temperature. For example: the ranking with the highest similarity can be regarded as the electroencephalogram file with the strongest emotion, the ranking with the lowest similarity can be regarded as the electroencephalogram file with the weakest emotion, and the electroencephalogram file with the strongest emotion can be regarded as the electroencephalogram file. As the "target value", the electroencephalogram file with the weakest emotion ranking is used as the "starting value". For the convenience of explanation, the most similar at least one electroencephalogram file is taken as the "target value", and the least similar at least one electroencephalogram file is taken as the "start value", and different scores are given, for example: "target value" is given 90 points for similarity, and 30 points for "initial value". Similarly, complete the electroencephalogram files of the series of happy emotions in the 4,000K color temperature category, and the electroencephalogram files of the series of the surprised emotions in the 5,700K color temperature category. Among them, the "start value" and "target value" can be formed into a score interval of similarity.

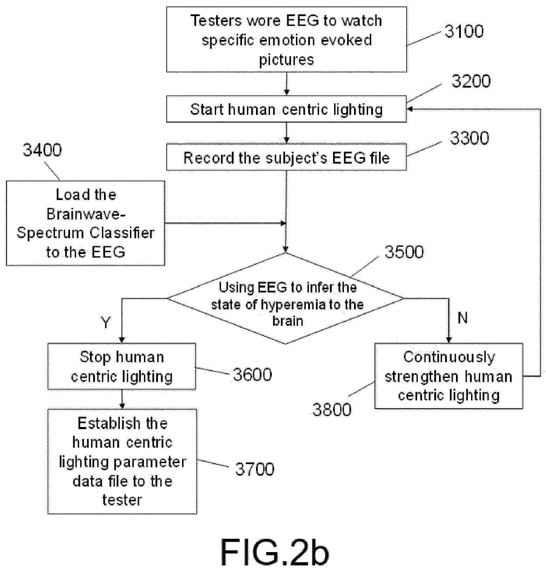

Afterwards, as shown in step 2500, an electroencephalogram classification and grading database in artificial intelligence (it may be referred to as an artificial intelligence electroencephalogram file database) is established. After the step 2400 is passed, the classification results of the "target value" score and the "start value" score are given to the electroencephalogram file groups of various specific color temperatures to form a database, which is stored in the memory of the EEG 130. The purpose of establishing the electroencephalogram classification and grading database in step 2500 of the present invention is to obtain the electroencephalogram file of the unknown tester after receiving a specific emotional stimulus and giving lighting of a specific color temperature to an unknown tester. After the similarity score interval between the electroencephalogram file of the unknown tester and the electroencephalogram file in the database is compared, it can be used to determine or infer the current state of the unknown tester's hyperemia reaction in the brain. The detailed process is shown in FIG.2b shown.

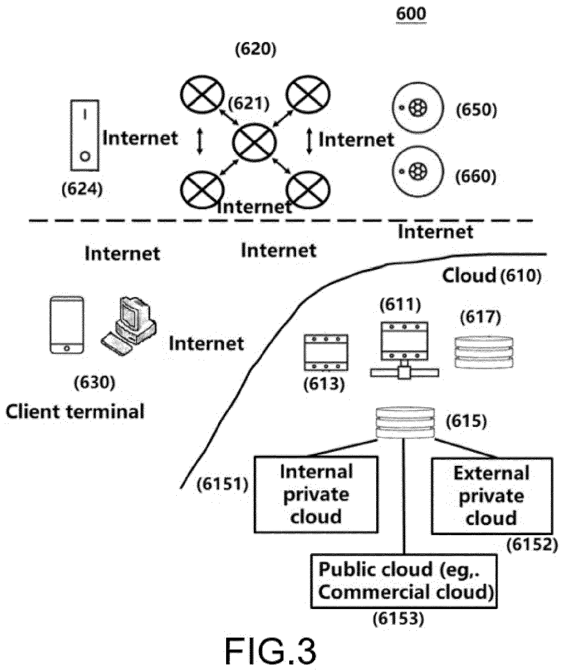

Next, please refer to FIG.2b, which is a lighting database for constructing a user's effective human centric lighting according to the present invention. First, as shown in step 3100, the tester is put on the EEG 130, and the tester is allowed to watch a picture evoked by a specific emotion. Afterwards, as shown in step 3200, the intelligent human centric lighting system 100 is activated to irradiate the tester with the light recipe by management control module 611 (as shown in FIG.3) in an environment (e.g., a test space) that has been configured with various adjustable multispectral lighting modules. To provide lighting parameters such as spectrum, light intensity, flashing rate, color temperature, exposure time and other light signal light parameters that can be changed. Next, as shown in step 3300, obtain and record the electroencephalogram file of the tester after the induced emotion and lighting, and store it in the memory module 617 of the cloud 610 (as shown in FIG.3). Next, as shown in step 3400, import the artificial intelligence electroencephalogram file database into the management control module 611. Wherein, the management control module 611 will set a score of whether the similarity is sufficient. For example, when the similarity score is set to be more than 75 points, it means that the tester's brain hyperemia reaction is sufficient. Next, as shown in step 3500, in the management control module 611, the similarity between the tester's electroencephalogram file and the artificial intelligence electroencephalogram file is compared. For example: when the similarity score of the tester's electroencephalogram files after comparison is 90 points, the management control module 611 immediately determines that the tester's brain hyperemia reaction is very sufficient, so it will go to step 3600 to terminate the person with a specific emotion Due to lighting test. Next, go to step 3700, record the human centric lighting parameters in the tester's brain when the hyperemia response has reached the stimulus, into a database and store in the memory module 617.

Next, in the procedure of step 3500 in FIG.2b, if the similarity score after the comparison between the tester's electroencephalogram file and the artificial intelligence electroencephalogram file is 35 points, the management control module 611 will determine the tester's brain If the hyperemia reaction is insufficient, step 3800 will be performed, and the management control module 611 will continue to strengthen the human centric lighting test, including: according to the similarity score, the management control module 611 can control it to provide an appropriate increase in the lighting time or increase light intensity. Then, the electroencephalogram file after increasing the lighting time or intensity is obtained again through step 3300. After step 3400, the human centric lighting test is not stopped until the similarity score reaches the set similarity score of more than 75 points. Wherein, when the management control module 611 determines that the hyperemia reaction in the tester's brain has reached the stimulation, in step 3700, a data file of the human centric lighting parameters of the tester is created. Finally, the management control module 611 will form a "human centric lighting parameter database" of the human centric lighting parameters of each tester and store it in the memory module 617. Obviously, when there are more testers, the artificial intelligence electroencephalogram file database of the present invention will learn more electroencephalogram files, so that the similarity score of the present invention is more and more accurate.

After the artificial intelligence model of the "human centric lighting parameter database" is established, after the intelligent human centric lighting system 100 is activated, the present invention can deduce the result only by observing the electroencephalogram file of the EEG 130, that is, it can be deduced The physiological and emotional changes in the brain images of the new test subjects can be inferred based on the artificial intelligence model of the "Human Centric Lighting Parameter Database" without using a high-value fMRI system. So that the intelligent human centric lighting system 100 can be promoted and used commercially. In addition, in order to enable the "human centric lighting parameter database" to be used commercially, the "human centric lighting parameter database" may be further stored in the internal private cloud 6151 in the cloud 610.

Next, please refer to FIG.3, which is a system architecture diagram of the intelligent human centric lighting system 100 of the present invention. As shown in FIG.3, the overall architecture of the intelligent human centric lighting system 100 of the present invention can be divided into three blocks, including: a cloud 610, a lighting field end 620 and a client terminal 630. The internet is used as a connection channel, so the three blocks can be distributed in different areas, and of course they can be configured together. The cloud 610 further includes: a management control module 611, which is used for cloud computing, cloud environment construction, cloud management or use of cloud computing resources, etc., and also allows users to access, construct or modify the content in each module through the management control module 611. The consumption module 613 is connected with the management control module 611 and is used as a cloud service subscribed and consumed by the user. Therefore, the consumption module 613 can access various modules in the cloud 610. The cloud environment module 615, connected with the management control module 611 and the consumption module 613, divides the cloud background environment into an internal private cloud 6151, an external private cloud 6153, and a public cloud 6155 (for example, a commercial cloud), etc., and can provide system providers or an interface to a user's external or internal service. The memory module 617 is connected to the management control module 611 and is used as a storage area of the cloud background. The technical contents required to be executed by each module in the present invention will be described in detail in the subsequent different embodiments. The lighting field end 620 can communicate with the cloud 610 or the client terminal 630 through the internet. The lighting field end 620 is provided with a LED lamps group 621 composed of a plurality of light-emitting devices. And the client terminal 630 can communicate with the cloud 610 or the lighting field end 620 through the internet. Among them, the client terminal 630 of the present invention includes general users and editors who use the intelligent human centric lighting system 100 of the present invention for various commercial operations, all of which belong to the client terminal 630 of the present invention, and the representative device of the client terminal 630 or the device can be a fixed device with computing function(including with edge operations) or a portable intelligent communication device. In the following description, the user, creator, editor or portable communication device can all represent the client terminal 630. In addition, in the present invention, the above-mentioned internet may be an Artificial Intelligence of Things (AIoT).

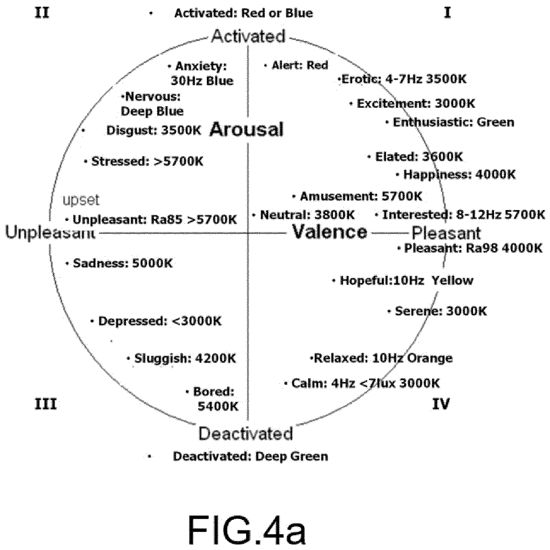

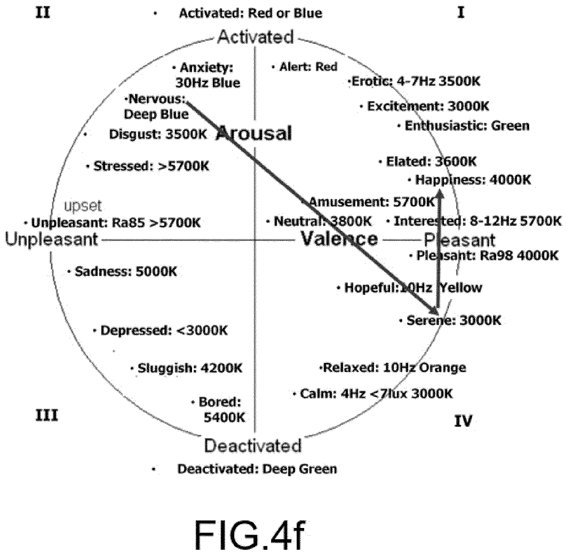

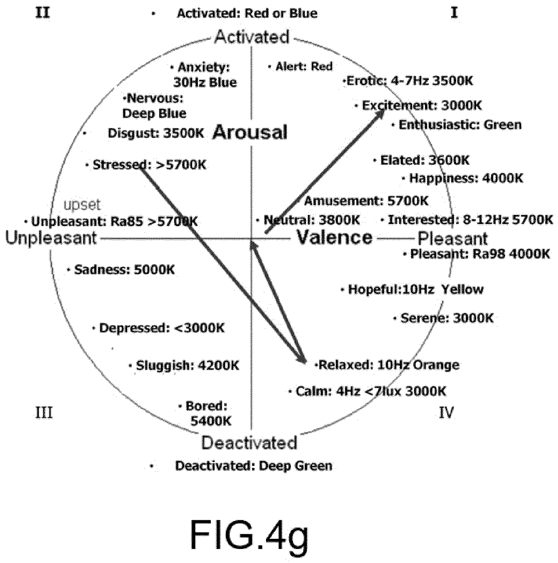

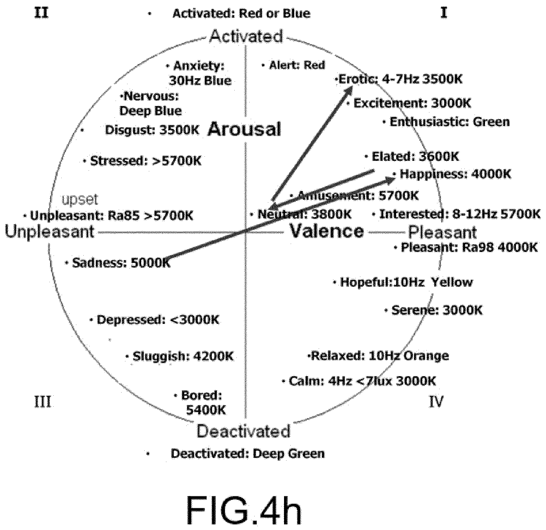

In a relatively complete electroencephalogram experiment process of the present invention, the tester is allowed to wear the EEG 130, in step 2300, the intelligent human centric lighting system 100 is activated to provide variable light signal parameters such as variable spectrum, light intensity(illuminance), flicker frequency, color temperature and general color rendering index (Ra) are stimulated to the tester. After light stimulation to the tester, through the results of the BOLD brain region test and the literature search, the light signal parameters of each emotion on the emotion coordinate system as shown in FIG.4a can be obtained, wherein the spectrum or optical signal parameters of some emotions on the emotion coordinate system are summarized as shown in Table 7 below.

Table 7

| I quadrant | | II quadrant | | III quadrant | | IV quadrant | |
|---|---|---|---|---|---|---|---|
| emotion | spectrum | emotion | spectrum | emotion | spectrum | emotion | spectrum |
| alert | red | nervous | Deep blue | sadness | 5000K | pleasant | Ra 98 4000k |
| excitement | 3000K | anxiety | 30Hz blue | depressed | <3000K | concentrate | 8-12Hz 4000k |
| elated | 3600K | stressed | > 5700K | sluggish | 4200K | relaxed | 10Hz orange |
| happiness | 4000K | upset | 4200K | bored | 5400K | calm | 4Hz < 7lux 3000K |

In Table 7, K refers to color temperature, lux refers to illuminance, Hz refers to flicker frequency, and Ra refers to color rendering (the full name is general color rendering index). In addition, a trend can be seen from FIG.4a and Table 7, that is, the emotions of the quadrant I area and the quadrant III area have complementary effects, and the emotions of the quadrant II area and the quadrant IV area also have a complementary effect. This phenomenon can be used as the navigation direction while emotion conversion.

Obviously, in order to construct a set of human centric lighting system and its method that can be operated commercially, it is necessary to use an expensive fMRI system to implement the human centric lighting system. The wave pattern is used to assist in determining the emotional change of the user, which can further meet the customized service requirements, and at the same time reduce the operating cost.

When performing emotion localization with fMRI systems and EEG, the emotion results obtained by the fMRI system are used as the standard basis. We define it as the peripheral system score (LSS), and the reason why the peripheral system score is used as an indicator is because the limbic system refers to the brain region that supports various emotions, behaviors, and long-term memory, including the hippocampus and amygdala. For example, the higher the BOLD index in fMRI, the bigger the emotion response. For example, when LSS can represent the arousal of emotion, the higher its value, the more excited the emotion. Therefore, the present invention will use LSS to establish the formulas combining light signal parameters such as various spectra, light intensity, flicker frequency, color temperature and color rendering (Ra), so as to define the light signal parameters as each emotion on the emotion coordinate system of FIG.4a.

According to the paper "fMRI BOLD Correlates of EEG Independent Components: Spatial Correspondence with the Default Mode Network" published in the Neuroscience Journal (Front. Hum. Neurosci) on November 27, 2018. Two important information are disclosed. One is that alpha waves have no significant impact on emotions, and the other is that the correlations of delta waves, theta waves, beta 2 waves, and gamma waves to blood oxygen responses in brain regions are -0.291, -0.26, 0.269, and -0.345, respectively.

Next, according to the number of brain regions corresponding to different brain waves in the present invention, there are 13, 18, 14, and 17 brain regions respectively. Wherein, the number of brain regions represents the probability of affecting the peripheral system (LSS), for example, the delta wave has affected 13 brain regions. In the equation 1 of the present invention, its probability is 13/(13+18+14+17) = 0.2097, so as the weight of delta should be adjusted from -0.291 to -0.291x0.2097=-0.0610. Calculating in the same way (theta=18/(13+18+14+17=0.2903, -0.26x0.2903=-0.0755), the equation 1 is obtained as follows, and the formula derives the relationship between light parameters such as flicker frequency (f), color rendering (Ra), color temperature (Color Temperature Index, CTI) and illuminance (I).

$$LSS = C_1\, delta - C_2\, theta + C_3\, beta2 - C_4\, gamma \qquad \text{equation 1}$$

Wherein, the coefficients C1=-0.061, C2=0.0755, C3=0.0607 and C4=0.0945

Next, the equation 1 is transformed into an emotional equation related to (f, Ra, CTI, I) in the EEG. First, if the color temperature index of a certain emotion obtained by the fMRI system is a certain value, then **fMRI(CTI) = LSS.**

Next, decompose the electroencephalogram into four optical parameter results, and make each optical parameter equal to the fMRI corresponding parameter.

$$f_{eeg}(Freq.) = LSS_{freq} = W_{freq} \times LSS$$
$$f_{eeg}(Ra) = LSS_{Ra} = W_{Ra} \times LSS$$
$$f_{eeg}(CTI) = LSS_{CTI} = W_{CTI} \times LSS \qquad \text{equation 2}$$
$$f_{eeg}(I) = LSS_{I} = W_{I} \times LSS$$

Wherein, 1/ Wfreq, 1/ WRa, 1/ WCTI, 1/ WI are the adjustment ratios of fMRI system and EEG for each optical parameter.

After the standardization process is carried out with $1/W_{CTI}$, the weight relationship (a, b, c, d) between the complete four coefficients of variation (a, b, c, d) can be obtained. After that, to final LSS formula is obtained as equation 3.

$$LSS = t \times [a \times f_{eeg}(freq) + b \times f_{eeg}(Ra) + c \times f_{eeg}(CTI) + d \times f_{eeg}(I)] \qquad \text{equation 3}$$

**a** = w_{freq}/w_{CTI}
**b** = w_{Ra}/w_{CTI}
**c** = w_{CTI}/w_{CTI} = 1
**d** = w_{I}/w_{CTI}
**t = illumination time**

It should be noted that when the illumination time (t) is greater, it means that the longer the user receives the illumination, the higher the emotion response will be. Therefore, the illumination time (t) is assumed to be 1 in the following description of the present invention.

According to the above formula, the light signal parameters of each emotion on the emotion coordinate system in FIG.4a can be obtained. For example, in a preferred embodiment under the excitement situation of the present invention, its LSS formula is as shown in following equation 4.

$$LSS = t \times (-0.0002\,freq^2 + 0.0393\,freq + 0.0334\,Ra - 0.2854\,CTI - 0.0000009\Delta I^2 + 0.0004\Delta I - 4.4441)$$

equation 4

In a preferred embodiment under the happiness situation of the present invention, its LSS formula is shown in the following equation 5.

$$LSS = t \times (-0.0002\,freq^2 + 0.0393\,freq + 0.0334\,Ra - 0.6374\,CTI - 0.0000009\Delta I^2 + 0.0004\Delta I - 4.4436)$$

equation 5

Wherein, $\Delta I^2$ is the change of illuminance.

According to the above LSS formula, the amount of emotion change under the condition of known various light parameters can be obtained.

LSS Example 1: If a lamp with 50Hz, Ra90, and color temperature of 6000K increases the illuminance by 200 lux within the visual range of the user, after a specific time of illumination, how much excitement does the user feel at this time? Apply the equation 4 and set t=1, freq=50 Hz, Ra=90, CTI=(6000-3000)/3000 =1, $\Delta I$=200, then get LSS = -0.1787, that is, the excitement decreased by 0.1787.

LSS Example 2: If a lamp with 60Hz, Ra95, and color temperature of 4000K increases the illuminance by 400 lux within the visual range of the user, after a specific time of illumination, how much excitement does the user feel at this time? Apply the equation 4 and set t=1, freq=60 Hz, Ra=95, CTI= (4000-3000)/3000 =0.33, $\Delta I$ = 400, then get LSS = 0.4324, which is an increase in excitement of 0.4324.

LSS Example 3: If a lamp with 60Hz, Ra=95, and color temperature of 4000K increases the illuminance by 400 lux within the visual range of the user, after a specific time of illumination, how happy is the user at this time? Apply Equation 5 and set t=1, freq=60 Hz, Ra=95, CTI=(4000-4000)/4000=0, $\Delta I$ = 400, then get LSS = 0.5270, which is an increase in happiness of 0.5270.

LSS Example 4: If a lamp with 50Hz, Ra=85, and color temperature of 2700K increases the illuminance by 400 lux within the visual range of the user, after a specific time of illumination, how happy is the user at this time? Apply the equation 5 and set t=1, freq=50 Hz, Ra=85, CTI=[(2700-4000)/4000]=0.325, $\Delta I$ = 400, then get LSS = -0.1871, that is, the sense of happiness has dropped by 0.1871.

A conclusion can be drawn from the calculation results of Example 1 and Example 2. To achieve the same level of excitement, there are many different means, such as increasing the color rendering (Ra), increasing the flicker frequency, increasing or reducing the illuminance (because there is an optimal value), and reducing the color temperature, etc. Because in the excitement situation, the higher the CTI, the lower the excitement. Conversely, on the other hand, by adjusting the color rendering (Ra), flicker frequency, illuminance and color temperature, you can also get different degrees of excitement. In addition, the same conclusion can also be obtained from the calculation results of Example 3 and Example 4. At this time, the light recipe of the lighting parameters such as color rendering (Ra), flicker frequency, illuminance and color temperature can also be called the multispectral recipe.

Obviously, Equation 3 is the equation of the multispectral formulation of the present invention. The equation 4 and the equation 5 are embodiments of the equation 3 corresponding to excitement and happiness in the present invention, but not used to limit the conditions of the equation 3. In other words, by controlling the "spectral recipe" such as color rendering (Ra), flicker frequency, illuminance and color temperature, different emotions, and different degrees of emotions in the emotional coordinates in FIG.4a or Table 5 can be combined. Therefore, when the lighting system of the present invention is in commercial operation, an experienced operator such as a professionally trained technician can operate in the lighting field according to the emotional and physiological conditions of the user. The client terminal 620 uses the software on the workstation or the portable smart device to adjust the characteristics of the lighting parameters in the LSS formula such as the equation 3 to edit the multispectral recipe for lighting to control the lighting system to execute the lighting program of the specific multispectral recipe at the lighting field end 620 to meet the requirement of users.

In a general emotion conversion method, when we perform the emotion conversion, we can perform the emotion conversion by giving the multispectral recipe of the desired emotion. For example, if we want to convert to an excitement emotion, a color temperature of 3000K can be directly given according to Table 5 or FIG.4a. Furthermore, for example, when the user is currently in a stressed emotion, the user's goal is to change the emotion to an excitement emotion. Intuitively, as long as the 3000K light is continuously given for a period of time, the user's emotion can reach the level of excitement. But this is inaccurate, because, according to the present invention under the condition of equation 3, it can be known that with regard to the same emotion, there can be different degrees of expression, and the different level of performance can be obtained through controlling parameters of multispectral recipe such as color rendering (Ra), flicker, illuminance and color temperature. In generally, when we want to perform emotion conversion, we can perform emotion conversion by giving the multispectral recipe of the emotion that we want to convert. For example, if you want to switch to happiness, according to Table 7 or Figure 4a, you can directly give a color temperature of 4000K. Furthermore, for example, when it is known that the user is currently in nervous, the user's goal is to change the emotion to happiness. Intuitively, as long as the 4000K light is continued for a period of time, the user's emotion can reach a level of happiness. But this is not accurate. Because, according to the present invention, it can be known under the condition of equation 3 that the same emotion can be expressed in different degrees. And this different level of performance can be adjusted and obtained by adjusting the lighting parameters of the multispectral recipe such as color rendering (Ra), flicker frequency, light intensity, and color temperature.

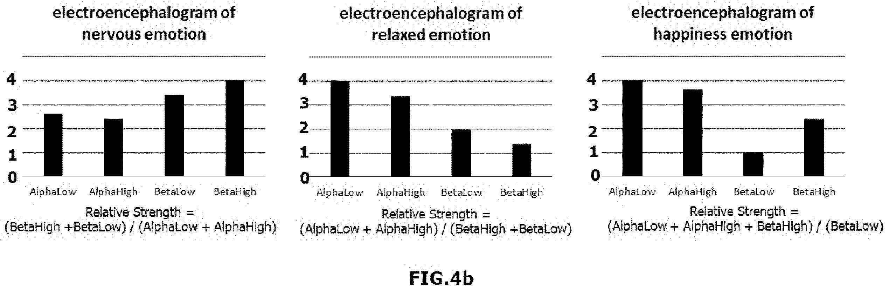

Next, the present invention can provide a method for effective emotion transfer through the following process. In the embodiment of the present invention, it is to allow the user to switch from nervous to happiness. This process is performed using the electroencephalogram measured by an EEG device. Wherein, the coordinate dimensions in FIG.4b to FIG.4e are normalized numbers.

As shown in FIG.4b, it is a relative strength index diagram of a specific electroencephalogram of the present invention. The relative strength index for the EEG was performed using the NeuroSky definition. Wherein, the definitions of brain wave waveforms and relative strength index related to emotions in this embodiment includes nervous, relaxed and happiness, as shown in FIG.4b. Wherein, the brain wave waveform diagram shown in FIG.4b is the high value of Beta wave, the low value of Beta wave, the high value of Alpha wave and the low value of Alpha wave under a specific situation. It should be noted that, in the following experiments, the values of the EEG of the same emotion may be different after different lighting stimuli are given. But the trend of the strength value of Beta wave and Alpha wave is similar. Wherein, the relative strength index of different emotions is calculated in different ways. Wherein, according to NeuroSky's definition of the relative strength index of the emotions of nervous, relaxed and happiness is as follows.

$$\text{Relative Strength of Nervous}=(\text{BetaHigh}+\text{BetaLow})/(\text{AlphaLow}+\text{AlphaHigh})$$

$$\text{Relative Strength of Relaxed}=(\text{AlphaLow}+\text{AlphaHigh})/(\text{BetaHigh}+\text{BetaLow})$$

$$\text{Relative Strength of Happiness}=(\text{AlphaLow}+\text{AlphaHigh}+\text{BetaHigh})/(\text{BetaLow})$$

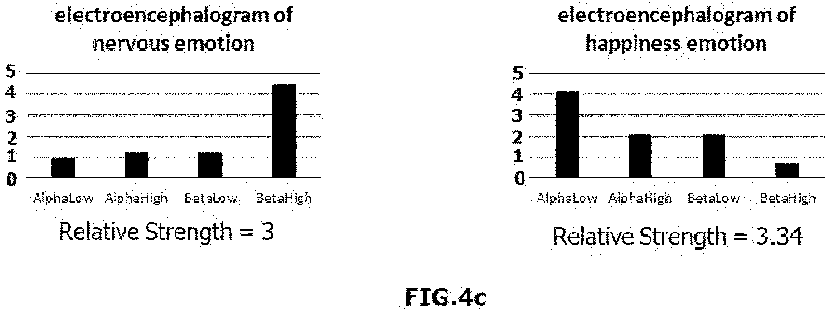

Next, please refer to FIG.4c, which is an electroencephalogram of the emotion conversion test 1 of the present invention. The first test for emotion conversion is to directly give a happiness color temperature of 4000K. The process is to first confirm that the user is already under nervous. For example, asking users to answer some math problems in full within 2 minutes may cause them to get nervous. When the user's electroencephalogram is obtained and the waveform shown on the left side of FIG.4c is obtained, it is determined that the user is already in nervous. Then, after directly irradiating the user with a color temperature of 4000K for 2 minutes, the user's electroencephalogram is obtained as shown on the right side of FIG.4c. Next, calculate the relative strength index of the happiness exhibited by the brain waves was 3.34.

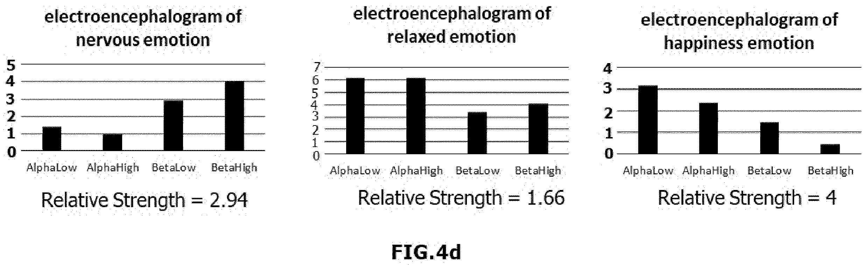

Next, please refer to FIG.4d, which is an electroencephalogram of the emotion conversion test 2 of the present invention. A second test of emotion conversion was conducted, allowing the user to go from nervous to relaxed to happiness. The process is to first confirm that the user is already under nervous. For example, asking users to answer some math problems in full within 2 minutes may cause them to get nervous. When the user's electroencephalogram is obtained and the waveform shown on the left side of FIG.4d is obtained, it is determined that the user is already in nervous. Then, give a relaxed spectrum (orange light of 10 Hz) irradiation for 5 minutes. Then, after irradiating the user with a color temperature of 4000K for 2 minutes, the user's electroencephalogram is obtained as shown in the middle and right side of FIG.4d. Next, calculate the relative strength index of the happiness exhibited by the brainwaves was 4.

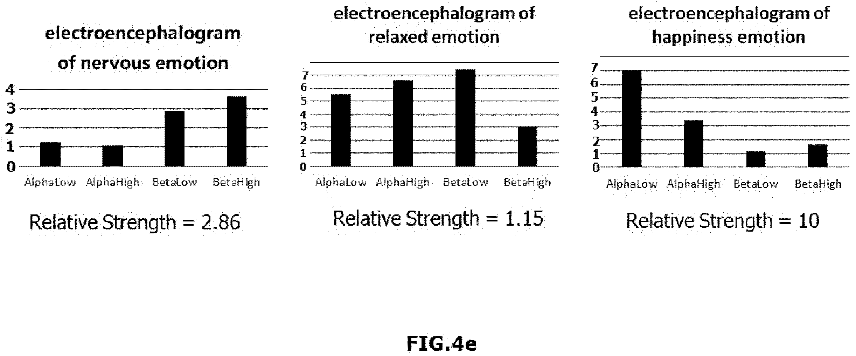

Next, please refer to FIG.4e, which is an electroencephalogram of the emotion conversion test 3 of the present invention. A third test of emotion conversion was performed, taking the user from nervous to neutral to happiness. The process is to first confirm that the user is already under nervous. For example, asking users to answer some math problems in full within 2 minutes may cause them to get nervous. When the user's electroencephalogram is obtained and the waveform shown on the left side of FIG.4e is obtained, it is determined that the user is already in nervous. They were then given a relaxed light spectrum (orange light of 10 Hz) exposure for 2 minutes to guide them into a neutral emotion by reducing nervous. For example, the relaxed EEG in the middle of FIG.4d shows that its relative strength index is 1.66, which is lower than the relative strength index of the second test process. Therefore, it is determined that the emotion state of the user has entered a neutral emotion at this time. Finally, after irradiating the user with a color temperature of 4000K for 2 minutes, the user's electroencephalogram is obtained as shown on the right side of FIG.4e. Next, a relative strength index of the happiness exhibited by the brain waves is calculated as 10.

According to the experimental processes mentioned above, one result can be obtained, that is, after confirming that the user is in nervous, in the first stage, some other spectrums that can ease the nervous emotion are first given. For example, in Table 7 or in FIG.4a, after some complementary emotion spectrum irradiations, the second stage of happiness spectrum irradiation can allow the user to obtain better index of happiness. In particular, after further confirming that the user has shifted emotion to a neutral emotion after the first stage of lightening to ease the nervous, the second stage of happiness spectrum irradiation can greatly improve the index of happiness. The theoretical neutral index means that the BOLD response of the limbic system returns to zero. Wherein, the neutral emotion is close to the center point or the origin of the emotion coordinate system in FIG.4a. Its theoretical feature is that it can keep ordinary people in a balanced and stable state of mind and will not be influenced by excessive positive or negative emotions, so that they can look at things and problems more objectively. Especially after the experimental process mentioned above, if the user can be allowed to go through the first stage of complementary emotion spectrum irradiation, and determine that the user's emotion has returned to neutral emotion. The best emotion conversion effect can be adjusted after being irradiated with the final emotion spectrum. And this confirms the current emotion, and then sets the first-stage irradiation spectrum according to the current emotion, and after determining that the user's emotion has reached a neutral emotion after the first-stage lighting, the second-stage final irradiation of the targeted emotion spectrum. And this kind of path or process of planning to reach the target emotion is called 「motion navigation」.

In addition, the present invention also finds that the change of emotion conversion is not linear. For example, when different users have different physiological conditions or social status, different users will have different reactions to the same emotion. For example, in terms of physiological conditions, users with epilepsy cannot be stimulated by flicker frequency, and must be reinforced in terms of color temperature or illuminance. For example, in different social status, the experience of happiness is different, which will cause different degrees of reflection. Therefore, a more rigorous way is that before we provide the lighting stimulation of the target emotion, we must consider the user's current emotion. The adjustment of the lighting parameters in the LSS formula should be given to different multispectral recipes stimuli in order to achieve the same final effect.

According to the emotion conversion test results, the present invention provides three different emotion conversion paths to perform the process of emotion navigation of the present invention.

Emotion Navigation Example 1

For example, the user is currently in a state of nervous, and the goal is to transfer emotions to happiness. The emotion navigation path of the transfer process can be set according to the experience of the user or the instructor.

First, it is necessary to ease the current nervous of the user so as to choose the serene multispectral recipe with a color temperature of 3000K for lighting according to Table 5. Then, choose the target emotion happiness multispectral recipe with a color temperature of 4000K for lighting according to Table 5. If it has reached the happiness of the emotion, the program of lighting is stopped. For the navigation path of emotion navigation Example 1, please refer to the FIG.4b.

Emotion Navigation Example 2

Emotion navigation example 2 is a preferred embodiment of the present invention, especially adding neutral indicators into the emotion navigation. For example, the user is currently in a state of stressed, and the goal is to transfer emotion to excitement. The emotion conversion process includes two steps.

The first step is to eliminate the stressed state first, to bring the user's emotion back to neutral or the origin point. Therefore, according to the emotion coordinate system in FIG.4a, the relative item of stressed is relaxed and it can choose the relative relaxed emotion item to complement the nervous emotion, so we should first give the relaxed "multispectral recipe" for lighting with 10Hz orange light according to Table 5. At the same time, it monitors the user's physiological signals (such as the response of the brain area), and determines that the user's emotion has returned to neutral or near the origin point. If it has returned to neutral or near the origin point, it means that the stress has been eliminated. Alternatively, it can be determined that the user's emotion has returned to neutral or near the original point by conducting interviews or questionnaires on the user.

The second step is to give the excitement multispectral recipe. After confirming that the user's emotion has returned to the neutral or the origin point, the excitement multispectral recipe is chosen for lighting with a color temperature of 3000K according to Table 5. At the same time, it monitors the user's physiological signals (such as the reaction of the brain area), and determines that the user's emotion has reached the emotion of excitement. If the user's emotion has reached the excitement, the lighting program will be stopped. For the navigation path of the emotion navigation Example 2, please refer to the FIG.4c.

Emotion Navigation Example 3

Emotion navigation example 3 is another preferred embodiment of the present invention. For example, the user is currently in a state of sadness, and the goal is to transfer emotion to erotic. The transfer process includes two steps.

The first step is to eliminate the sadness state first, to bring the user's emotion back to neutral or the origin point. Therefore, according to the emotion coordinate system in FIG.4a, it can choose the relative happiness emotion item to complement the nervous emotion, so we should first give the happiness multispectral recipe for lighting with a color temperature of 4000K according to Table 5. At the same time, it monitors the user's physiological signals (such as the response of the brain area), and determines that the user's emotion has returned to neutral or near the origin point. If it has returned to neutral or near the origin point, it means that the sadness has been eliminated.

The second step is to give the erotic multispectral recipe. After confirming that the user's emotion has returned to the origin point, the erotic multispectral recipe is chosen for lighting with a flicker frequency of 4-7Hz and a color temperature of 3500K according to Table 5. At the same time, it monitors the user's physiological signals (such as the reaction of the brain area), and determines that the user's emotion has reached the emotion of erotic. If the user's emotion has reached the erotic, the lighting program will be stopped. For the navigation path of the emotion navigation Example 3, please refer to the FIG.4d.

The above are all examples of the emotion navigation disclosed by the present invention, and its purpose is to explain the concept of the emotion navigation implementation of the present invention. It may be necessary to navigate through multiple emotion transitions to navigate to the target emotion. At this time, the goal of emotion navigation can be achieved by adjusting color rendering (Ra), flicker frequency, illuminance, and color temperature. Therefore, the present invention does not limit on how many emotion conversions can be performed to reach the target emotion.

Next, the present invention will further disclose preferred embodiments that can be specifically operated.

According to the above description, obviously, when the present invention performs emotion navigation, it is necessary to know the user's the current emotion state before the emotion conversion, as well as the emotion change situation during the emotion navigation process, and whether to finally reach the user's emotion state. The target emotion must be recorded with a physiological monitoring device in these stages, as a basis for modifying the emotion navigation path. Wherein, the physiological monitoring device described in the present invention may be an EEG or a wearable electronic device, such as a mobile phone or a wearable device, etc. Current emotion can be stimulated by using the emotion picture library, such as International Affection Picture System (IAPS).

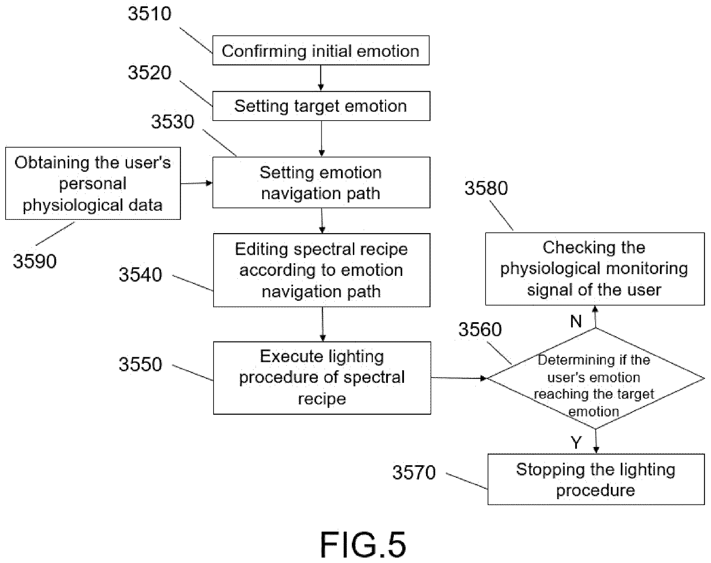

Please refer to FIG.5, which is a method for implementing emotion navigation in the present invention. First, as shown in step 3510, the initial emotion of the user is confirmed. For example, the user is located in the lighting field end 620, and after measuring the brainwaves through the wearable EEG 130, the state of the user's currently initial emotion is displayed according to the brainwave record data. For example, the initial emotion is in a state of nervous. Wherein, the initial emotion has been uploaded by the EEG 130 and stored in the memory module 617 of the cloud 610 (as shown in FIG.3). Next, it performs step 3520.

Step 3520 is to set the target emotion. When the user wants to adjust or convert the initial emotion to happiness emotion, the user sets the target emotion as happiness according to the demand of the client terminal 630 in the intelligent human centric lighting system 600, and then uploads and stores it in the memory module 617 of the cloud 610 through the client terminal 630. Next, go to step 3530.

Step 3530 is to choose the relay emotion to complete setting the emotion navigation path. According to the user's initial emotion and target emotion, select one or more relay emotions that are different from initial emotions or target emotions to form a sequence from initial emotions to relay emotions to target emotions by the client device 630 to the management control module 611 of the cloud 610. This sequence is called the emotion navigation path. For example, when the user's initial emotion is in a nervous state, and the "target emotion" is happiness, it can select the serene as the relay emotion by the client terminal 630 to the management control module 611 of the cloud 610, so as the emotion navigation path is from nervous to serene to happiness. On the other hand, it can select the calm as the relay emotion, so as the emotion navigation path is from nervous to calm to happiness. The relay emotion of the emotion navigation paths mentioned above can be selected by the user's own experience, or professionals can select the relay emotion based on the user's initial emotion and target emotion. Next, it proceeds to step 3540.

Step 3540 is to edit the multispectral recipe according to the emotion navigation path. After the emotion navigation path has been set in step 3530, it is necessary to find out the corresponding multispectral recipe of the relay emotion and the target emotion. For example, when the emotion coordinate system in FIG.4a or Table 5 has been stored in the memory module 617 of the cloud 610, the user can find out the multispectral recipe corresponding to the relay emotion and the target emotion in the cloud environment module 615 through the client terminal 630 in the intelligent human centric lighting system 600. The multispectral recipe includes the serene multispectral recipe (color temperature of 3000K), the calm multispectral recipe (flicker frequency is 4 Hz, illuminance is less than 7 lux 3000K color temperature), and the happiness multispectral recipe (color temperature of 4000K). Wherein, the client terminal 630 may be a smart phone, a personal digital assistant (PDA), a notebook computer (NB), or a personal computer (PC) in a workstation, and the like.

After that, go to step 3550. In particular, it should be noted that in the process of editing the multispectral recipe above, the multispectral recipe for performing lighting is edited by adjusting the characteristics of the lighting parameters in the surrounding system score (LSS) formula one by one.

Step 3550 is to execute the lighting procedure of the multispectral recipe. When executing the lighting program, the intelligent human centric lighting system 600 of the present invention can be controlled by two control paths. One of the control paths is to use the user terminal device 630 to control the lamps group 621 in the lighting field end 620 to sequentially illuminate the user according to the multispectral recipe corresponding to the relay emotion and the target emotion. Another control path is to use the management control module 611 in the cloud 610 to control the lamps group 621 in the lighting field end 620 through the intelligent Internet of Things (AIoT) according to the corresponding relay emotion and target emotion. For example, the two control ways are to irradiate the multispectral recipe of the user's relay emotion for 10 minutes, and then irradiate the multispectral recipe of the user's target emotion for 15 minutes.

Step 3560 is to determine if the user's emotion has reached the target emotion. If it is determined that the user's emotion has reached the target emotion, it is to execute step 3570. If it is determined that the user's emotion has not reached the target emotion, it is to execute step 3580.

Step 3570 is to stop the lighting procedure. If it is determined that the user's emotion has reached the target emotion, the lighting procedure is stopped. Wherein, the determination described in step 3560 is determined according to the information displayed by the physiological monitoring device configured on the user, wherein the physiological monitoring device can be an EEG or a wearable device that can detect sympathetic and parasympathetic nerve signals.

Step 3580 is to check the physiological monitoring signal of the user. If it is determined that the user does not reach the target emotion, it can be adjusted by checking the physiological monitoring signal displayed on the physiological monitoring device configured on the user's body. The adjustment of the segment that does not reach the set emotion state can be carried out through the management control module 611 of the client terminal 630 to the cloud 610 according to the emotion navigation path to determine which segment does not reach the set emotion (that is, the relay emotion segment or the target emotion segment). Then, reset a new emotion navigation path. For example, the original set emotion navigation path is from calm to serene, then check the lighting range of the physiological monitoring device from calm to serene, if calm or serene has not reached the set emotions, then adjust these emotions. The adjustment method can be through the management control module 611 to select a new relay emotion near the unaccomplished emotion. For example, if the result of the inspection is that calm is the unaccomplished emotion, then re-select relaxed as the new relay emotion and repeat step 3530 to step 3560. After returning to step 3530, a new emotion navigation path will be formed, from nervous to relaxed to serene, and finally to happiness. Finally, until it is determined that the user's emotion has reached the target emotion, go to step 3570, and stop the lighting procedure. In a preferred embodiment, selecting a new relay emotion can adjust the lighting parameters (such as Ra, flicker, illuminance, or color temperature, etc.) of the multispectral recipe through the aforementioned equation 3 in the management control module 611 of the client device 630 or the cloud 610. For example, increasing the Ra can make the calm emotion stronger.

In addition, if step 3580 has already determined which segment does not reach the set emotion through the physiological monitoring device, then the client terminal 630 can go to step 3590 to obtain the user's personal physiological data stored in the cloud 610, such as the physiological data of the user's health checkup. For example, according to the physiological data of the health checkup, when the user suffers from high blood pre4540ssure, diabetes, or epilepsy, it can adjust the lighting parameters (such as Ra, flicker, illuminance, or color temperature, etc.) of the multispectral recipe in the equation 3 according to these diseases. For example, when the user suffers from epilepsy, since stroboscopic flicker will stimulate the epilepsy, which may induce epilepsy. Hence, in equation 3, reinforcement must be given on the Ra, color temperature or illuminance. Next, repeat step 3530 to step 3560 to find a new relay emotion that can be adjusted to the target emotion based on the user's physiological signal data, and form a new emotion navigation path until the user's emotion is determined reaching the target emotion, then go to step 3570 and stop the lighting procedure.

It should be emphasized that the present invention uses the physiological data of the health check to adjust the lighting parameters (color rendering property-Ra, flicker frequency, illuminance, or color temperature) of the multispectral recipe in the equation 3. The limit of the lighting parameters of the multispectral recipe corresponding to each mentioned disease can be adjusted according to medical information. For example, a too low color temperature will affect the clarity of the user's vision. Therefore, when users need exciting stimuli in the working environment, it is not the best way to lower the color temperature but should be supplemented by other parameter items, such as to give higher color rendering (Ra) to compensate for the need to use too low color temperature parameters to maintain excitement to a certain extent. Therefore, in the above-mentioned embodiments of the present invention, the limitation use of flicker frequency for epilepsy patients is just an example, and it is not used to limit the present invention through the lighting parameter adjustment of the multispectral recipe in equation 3, only limited to the embodiment of epilepsy.

Furthermore, since the intelligent human centric lighting system 600 of the present invention uses the AIoT to communicate between the client terminal 630 and the cloud 610. Therefore, the emotion navigation paths used by many users will be stored in the cloud environment module 615, forming massive data. For example, after the massive data stored in the private cloud 6151 or the public cloud 6153 is processed by the algorithm of artificial intelligence, the ranking of the emotion navigation paths used by the most people in the process of various emotion conversions can be obtained. Therefore, when the current user wants to perform a specific emotion conversion, he can go to the private cloud 6151 or the public cloud 6153 through the client terminal 630 to find the emotion navigation path used by the most people among the data of the specific emotion conversion as a shortcut path for providing step 3530 to complete setting the emotion navigation path. Obviously, since the emotion navigation path of the present invention is operated through a network platform and also provides public cloud services. Therefore, the intelligent human centric lighting system 600 of the present invention is very suitable for various professionals to get better emotion navigation path services through the cloud platform.

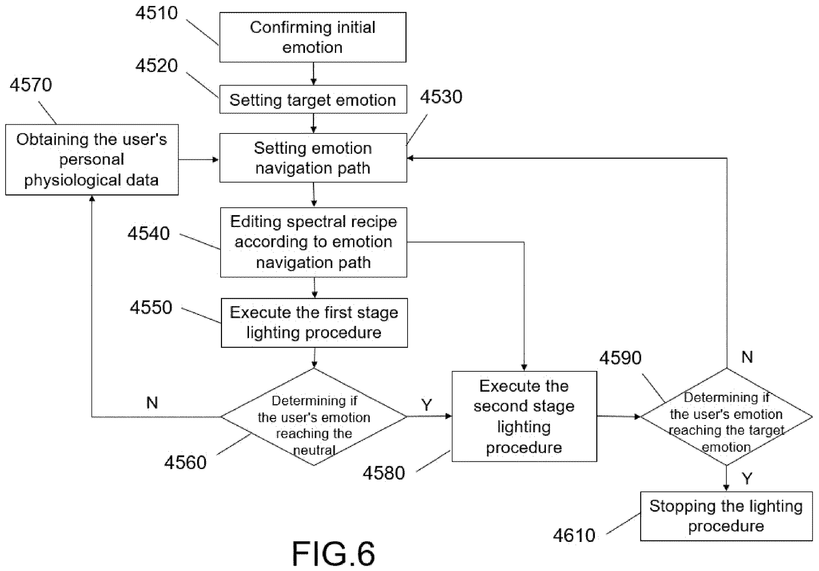

Please refer to FIG.6, which is another method for implementing emotion navigation in the present invention.

First, execute step 4510 to confirm the initial emotion. The detailed process is the same as step 3510, so please refer to the description of step 3510. Wherein, the initial emotion is in a state of stressed. Next, proceed to step 4520.

Step 4520 is to set the target emotion. The detailed process is the same as step 3520, so please refer to the description of step 3520. Wherein, the target emotion is set to excitement. Next, go to step 4530.

Step 4530 is to choose the relay emotion to complete setting the emotion navigation path. In this embodiment, step 4530 is to set the emotion navigation path according to the process of the emotion navigation Example 2 and the emotion navigation Example 3. Using the emotion navigation example 2 as an example, after the user has confirmed that the initial emotion is in a stressed state in step 4510. In step 4520, the user has also confirmed that the target emotion is excitement. The first step is to eliminate the state of stress to bring the user's emotion back to neutral point or the origin point. Therefore, in this embodiment, according to the emotion coordinate system shown in FIG.4a, the relaxed emotion corresponding to stressed is selected as the relay emotion of this embodiment. At this time, the emotion navigation path completed in this step is from the stressed of the initial emotion to the relaxed of the relay emotion and then to the excitement of the target emotion. Of course, the relay emotion in this embodiment can also be a combination of more than one different emotion. For example, the relay emotion can first select the corresponding calm emotion, and then further select the relaxed emotion. Therefore, the setting of the emotion navigation path in this embodiment is from the stressed of the initial emotion to the calm and relaxed of the relay emotion and then to the excitement of the target emotion. The present invention does not limit the number of combinations of relay emotion. Next, proceed to step 4540.

Step 4540 is to edit the multispectral recipe according to the emotion navigation path. After the emotion navigation path has been confirmed in step 4530, it is necessary to find out the corresponding multispectral recipe of relay emotion and target emotion. For example, when the emotion coordinate system in FIG.4a or Table 5 has been stored in the cloud environment module 615, the user can find out the multispectral recipe corresponding to the relay emotion and the target emotion in the cloud environment module 615 through the client terminal 630. The multispectral recipe includes the relaxed multispectral recipe (orange light with a flicker frequency of 10Hz) and the excitement multispectral recipe (color temperature of 3000K). Wherein, the client terminal 630 may be a smart phone, a personal digital assistant (PDA), a notebook computer (NB), or a personal computer (PC) in a workstation, and the like. After that, go to step 4550. In particular, in the process of editing the multispectral recipe in step 4540, the multispectral recipe for performing lighting is edited by adjusting the characteristics of the lighting parameters in the surrounding system score (LSS) formula one by one.

Step 4550 is to execute the first stage lighting procedure. According to the emotion navigation path set in step 4530, the user uses the client terminal 630 or the management control module 611 in the cloud 610 to control the lamps group 621 in the lighting field end 620 by the AIoT. In this embodiment, the first stage lighting is to execute the lighting procedure of the relaxed multispectral recipe (orange light with a flicker frequency of 10 Hz). For example, after the user is irradiated with the relaxed multispectral recipe for 10 minutes, the lighting program is stopped so as to complete the first stage lighting. If the relay emotion is a combination of more than one different emotion, it is necessary to irradiate all the different emotions multispectral recipe to the user before stopping the first stage lighting procedure. After that, proceed to step 4560.

Step 4560 is to determine if the user's emotion has reached the neutral point. In this step, it is to determine whether the stressed emotion of the user has been eliminated. According to the physiological monitoring device, after the user has passed the first stage lighting procedure, it is determined whether the user's current mood has been adjusted to the neutral point or the original point. If it is determined that the user's emotion has not reached the neutral point or the original point of FIG.4a, it indicates that the stressed emotion has not been completely eliminated so as it is to execute step 4570. If it is determined that the user's emotion has reached the region of the neutral point or the original point of FIG.4a, it is to execute step 4580.

Step 4570 is to obtain the user's personal physiological data. If it is determined that the user's emotion has not reached the neutral or origin point, the user's personal physiological data, such as health checkup data, can be further use to adjust the relay emotion. In a preferred embodiment, the client terminal 630 obtains the user's personal physiological data stored in the memory module 617 through the cloud 610. Afterwards, according to the information on the user's personal physiological data, adjust the lighting parameters in the LLS formula of the relay emotion. For example, according to the physiological data of the health checkup, when the user suffers from high blood pressure, diabetes or epilepsy, the lighting parameters (Ra, flicker frequency, illuminance, or color temperature, etc.) of the multispectral recipe in the equation 3 can be adjusted according to these diseases. Then, return to step 4530 to form a new emotion navigation path. After that, step 4550 and step 4560 are repeatedly executed until it is determined that the user's emotion has reached the neutral or origin point, then step 4580 is performed.

Step 4580 is to execute the second stage lighting procedure. According to the multispectral recipe of the emotion navigation path set in step 4540, through the client terminal 630 or the management control module 611 in the cloud 610, and then through the AIoT to control the lamps group 621 in the lighting field end 620 to execute the lighting program of the excitement multispectral recipe (3000K color temperature). For example, after the user is irradiated with the excitement multispectral recipe for 15 minutes, the lighting program is stopped so as to complete the second stage lighting. After that, proceed to step 4590.

Step 4590 is to determine if the user's emotion reaching the target emotion. If it is determined that the user's emotion has reached the target emotion, it is to execute step 4610. If it is determined that the user's emotion has not reached the target emotion, it is to execute step 4530 and then the client terminal 630 can go to step 4570 to obtain the user's personal physiological data stored in the cloud 610. After that, according to the information on the user's personal physiological data, adjust the parameters in the multispectral recipe of the target emotion. For example, according to the physiological data of the health checkup, when the user suffers from high blood pressure, diabetes, or epilepsy, it can adjust the lighting parameters (such as Ra, flicker, illuminance, or color temperature, etc.) of the multispectral recipe tin the equation 3 according to these diseases and the lighting parameters store in the cloud 610. Next, go to step 4580 to find out the adjusted lighting parameter of multispectral recipe of the target emotion in the cloud 610 through the client terminal 630. Next, repeat step 4580 to step 4590 until it is determined that the user's emotion has reached the target emotion, then go to step 4610 to stop the lighting procedure.

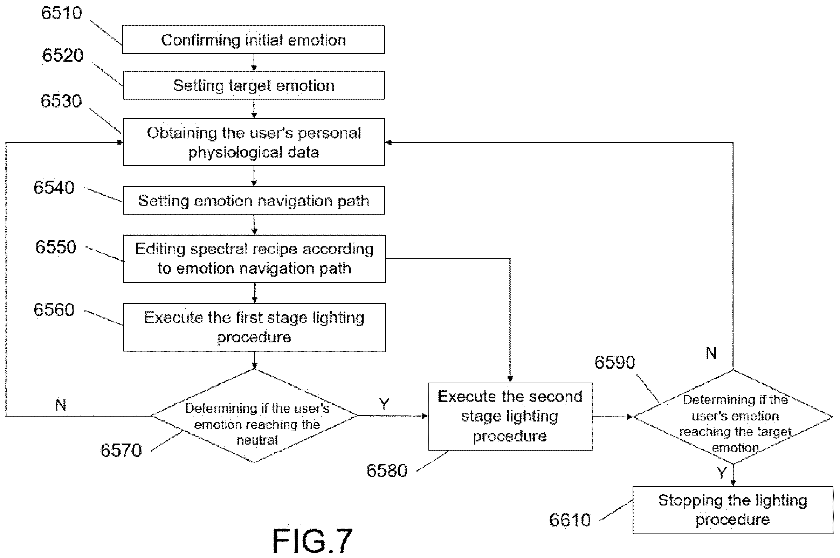

Please refer to FIG.7, which is the other method of implementing emotion navigation in the present invention.

First, execute step 6510 to confirm the initial emotion. The detailed process is the same as step 4510, so please refer to the description of step 4510. Wherein, the initial emotion is in a state of stressed. Next, proceed to step 6520.

Step 6520 is to set the target emotion. The detailed process is the same as step 4520, so please refer to the description of step 4520. Wherein, the target emotion is set to excitement. Next, go to step 6530.

Step 6530 is to obtain the user's personal physiological data. The client terminal 630 obtains the user's personal physiological data stored in the memory module 617 from the cloud 610, such as the physiological data of the user's health check. Next, go to step 6540.

Step 6540 is to choose the relay emotion to complete setting the emotion navigation path. In this embodiment, step 6540 is to set the emotion navigation path according to the process of the emotion navigation Example 2. After the user has confirmed that the initial emotion is in a stressed state in step 6510. In step 6520, the user has also confirmed that the target emotion is excitement. Next, the user chooses to set the emotion navigation path in the management and control module 611 of the cloud 610 through the client terminal 630. The first step is to eliminate the state of stress to bring the user's emotion back to neutral or the origin point. Therefore, in this embodiment, according to the emotion coordinate system shown in FIG.4a, the relaxed emotion corresponding to stressed is selected as the relay emotion of this embodiment. In this embodiment, the management control module 611 of the cloud 610 will also check the user's personal physiological data at the same time and adjust the multispectral recipe of the relay emotion according to the information on the user's personal physiological data. For example, when the user suffers from epilepsy, since flicker frequency can stimulate epilepsy, which may induce epilepsy, in Equation 3, the ratio of flicker frequency must be reduced or removed, so it must be in the Ra, color temperature or lighting intensity are reinforced to create a new relay emotion. At this point, the emotion navigation path completed in this step is from the stressed emotion of the initial emotion to the adjusted relaxed emotion of the relay emotion and then to the excitement of the target emotion. Wherein, the adjusted lighting parameters of the multispectral recipe for relaxed emotion are adjusted through the user's personal physiological data. Next, go to step 6550.

Step 6550 is to edit the multispectral recipe according to the emotion navigation path. After the emotion navigation path has been confirmed in step 6530, it is necessary to find out the corresponding multispectral recipe of relay emotion and target emotion. For example, when the emotion coordinate system in FIG.4a or Table 5 has been stored in the cloud environment module 615, the user can find out the multispectral recipe corresponding to the relay emotion and the target emotion in the cloud environment module 615 through the client terminal 630. The multispectral recipe includes the relaxed multispectral recipe (orange light with a flicker frequency of 10Hz) and the excitement multispectral recipe (color temperature of 3000K). Wherein, the adjusted relaxed multispectral recipe is to adjust the lighting parameters of the multispectral recipe such as Ra, illuminance, or color temperature in the equation 3. The client terminal 630 may be a smart phone, a personal digital assistant (PDA), a notebook computer (NB), or a personal computer (PC) in a workstation, and the like. After that, go to step 6560. In particular, in the process of editing the multispectral recipe in step 6550, the multispectral recipe for performing lighting is edited by adjusting the characteristics of the lighting parameters in the surrounding system score (LSS) formula one by one.

Step 6560 is to execute the first stage lighting procedure. After finishing the editing of the multispectral recipe of the emotion navigation path in step 6550, the user uses the client terminal 630 or the management control module 611 in the cloud 610 to control the lamps group 621 in the lighting field end 620 by the AIoT. In this embodiment, the first stage lighting is to execute the lighting procedure of the adjusted relaxed multispectral recipe. For example, after the user is irradiated with the relaxed multispectral recipe for 15 minutes, the lighting program is stopped so as to complete the first stage lighting. If the relay emotion is a combination of more than one different emotion, it is necessary to irradiate all the different emotions multispectral recipe to the user before stopping the first stage lighting procedure. After that, proceed to step 6570.

Step 6570 is to determine if the user's emotion has reached the neutral. In this step, it is to determine whether the stressed emotion of the user has been eliminated. According to the physiological monitoring device, after the user has passed the first stage lighting procedure, it is determined whether the user's current mood has been adjusted to the neutral or original point. If it is determined that the user's emotion has not reached the neutral or original point of FIG.4c, it indicates that the stressed emotion has not been completely eliminated so as it is to execute step 6530. If it is determined that the user's emotion has reached the neutral point or the original point of FIG.4c, it is to execute step 6580.

If it is determined that the user's emotion has not reached the neutral or origin point in step 6570, return to step 6530 to obtain the user's personal physiological data to adjust the relay emotion. In a preferred embodiment, the client terminal 630 obtains the user's personal physiological data stored in the memory module 617 through the cloud 610. Afterwards, according to the information on the user's personal physiological data, adjust the lighting parameters in the LLS formula of the relay emotion. For example, according to the physiological data of the health checkup, when the user suffers from high blood pressure or diabetes, the lighting parameters (Ra, flicker frequency, illuminance, or color temperature, etc.) of the multispectral recipe in the equation 3 can be adjusted according to these diseases. Then, step 6540 and step 6570 are repeatedly executed until it is determined that the user's emotion has reached the neutral or origin point, then step 6580 is performed.

Step 6580 is to execute the second stage lighting procedure. According to the multispectral recipe of the emotion navigation path set in step 6550, the user uses the client terminal 630 or the management control module 611 in the cloud 610 to control the lamps group 621 in the lighting field end 620 to execute the lighting program of the excitement multispectral recipe (3000K color temperature) by the AIoT. For example, after the user is irradiated with the excitement multispectral recipe for 15 minutes, the lighting program is stopped so as to complete the second stage lighting. After that, proceed to step 6590.

Step 6590 is to determine if the user's emotion reaching the target emotion. If it is determined that the user's emotion has reached the target emotion, it is to execute step 6610. If it is determined that the user's emotion has not reached the target emotion, it is to execute step 6530 to obtain the user's personal physiological data stored in the cloud 610 by the client terminal 630. After that, according to the information on the user's personal physiological data, adjust the lighting parameters in the multispectral recipe of the target emotion. For example, according to the physiological data of the health checkup, when the user suffers from high blood pressure, diabetes, or epilepsy, it can adjust the lighting parameters (such as Ra, flicker, illuminance, or color temperature, etc.) of the multispectral recipe through the equation 3 according to these diseases to obtain the adjusted light parameter of multispectral recipe of the target emotion and the lighting parameters store in the cloud 610. Next, go to step 6550 to get the adjusted multispectral recipe of the target emotion to go to step 6580. Next, repeat step 6590 to step 6530 to step 6550 and from step 6550 direct to step 6580 until it is determined that the user's emotion has reached the target emotion in step 6590, then go to step 6610 to stop the lighting procedure.

Step 6610 is to stop the lighting procedure.

Finally, it should be emphasized that the present invention uses the physiological data of the health check to adjust the parameters (color rendering property-Ra, flicker frequency, illuminance, or color temperature) of the multispectral recipe in Equation 3. The limitations of the parameters of the multispectral recipe corresponding to each disease described in it can be adjusted according to medical information. For example, a too low color temperature will affect the clarity of the user's vision. Therefore, when users need exciting stimuli in the working environment, it is not the best way to lower the color temperature but should be supplemented by other parameter items, such as to give higher color rendering (Ra) to compensate for the need to use too low color temperature parameters to maintain excitement to a certain extent. Therefore, in the above-mentioned embodiments of the present invention, the limitation use of flicker frequency for epilepsy patients is just an example, and it is not used to limit the present invention through the parameter adjustment of the multispectral recipe in equation 3, only limited to the embodiment of epilepsy.

Finally, it should be emphasized again that the above description is only a better embodiment of the invention and is not intended to limit the scope of the invention. At the same time, the above description can be understood and implemented by those who have ordinary knowledge in the relevant technical field. Therefore, other equivalent changes or modifications not divorced from the concepts disclosed in the invention should be included in the scope of patent claims of the invention.

**Claims**

1. A method for implementing emotion navigation by using an intelligent human centric lighting system, wherein the intelligent human centric lighting system is composed of a cloud, a lighting field end and a client terminal which are connected to each other through the Internet and an emotion coordinate information is stored in the cloud, and wherein the emotion navigation method including:

   step 1 : confirming an initial emotion of a user through a physiological monitoring device or by using an International Affection Picture System (IAPS) to simulate for confirming the initial emotion of the user, and storing the initial emotion in the cloud;
   step 2 : setting a target emotion according to a requirement of the user by the client terminal, and storing the target emotion in the cloud;
   step 3 : choosing a relay emotion to set an emotion navigation path and storing the relay emotion in the cloud, wherein the client terminal is connected the cloud through the Internet and the relay emotion is chosen from the emotion coordinate information in the cloud;
   step 4 : editing a multispectral recipe according to the light recipe of the relay emotion and the light recipe of the target emotion;
   step 5 : performing a lighting program, wherein the client terminal control a lamps group in the lighting field end to sequentially conducting a lighting process to the user for a set time according to the multispectral recipe corresponding to the relay emotion and the multispectral recipe corresponding to the target emotion;
   step 6 : determining if the user's emotion reaching the target emotion through the physiological monitoring device; and
   step 7 : stopping the lighting program when the user's emotion is reaching the target emotion.

2. The emotion navigation method to claim 1, wherein the physiological monitoring device can an EEG, a mobile phone or a wearable device that can detect sympathetic and parasympathetic nerve signals.

3. The emotion navigation method to claim 1, wherein the multispectral recipe includes lighting parameters such as color temperature, illuminance, flicker frequency, and color rendering (Ra).

4. The emotion navigation method to claim 3, wherein the multispectral recipe is a formula formed by the surrounding system score (LSS).

$$LSS = t \times [a \times f_{eeg}(freq) + b \times f_{eeg}(Ra) + c \times f_{eeg}(CTI) + d \times f_{eeg}(I)]$$

$a = w_{freq}/w_{CTI}$
$b = w_{Ra}/w_{CTI}$
$C = w_{CTI}/w_{CTI} = 1$
$d = w_I/w_{CTI}$
$t$ = illumination time

5. The emotion navigation method to claim 1, wherein another relay emotion is selected if the user's emotion is not reaching the target emotion.

6. The emotion navigation method to claim 5, wherein after selecting another relay emotion, step 3 to step 6 are re-executed.

7. The emotion navigation method to claim 1, wherein further includes a step 8 to obtain a personal physiological data of the user if the determination of the user's emotion is not reaching the target emotion.

8. The emotion navigation method to claim 7, wherein the personal physiological data is the physiological data of the user's health checkup.

9. A method for implementing emotion navigation by using an intelligent human centric lighting system, wherein the intelligent human centric lighting system is composed of a cloud, a lighting field end and a client terminal which are connected to each other through the Internet and an emotion coordinate information is stored in the cloud, and wherein the emotion navigation method including:

step 1 : confirming an initial emotion of a user through a physiological monitoring device or by using an International Affection Picture System (IAPS) to simulate for confirming the initial emotion of the user, and storing the initial emotion in the cloud.

step 2 : setting a target emotion according to a requirement of the user by the client terminal, and storing the target emotion in the cloud;

step 3 : choosing a relay emotion to set an emotion navigation path and storing the relay emotion in the cloud, wherein the client terminal is connected the cloud through the Internet and the relay emotion is chosen from an emotion coordinate information in the cloud;

step 4 : editing a multispectral recipe according to the light recipe of the relay emotion and the light recipe of the target emotion to edit the multispectral recipe corresponding to the relay emotion and the multispectral recipe corresponding to the target emotion;

step 5 : performing a lighting program of the multispectral recipe corresponding to the relay emotion, wherein the client terminal control a lamps group in the lighting field end to conducting a lighting process to the user for a set time according to the multispectral recipe corresponding to the relay emotion;

step 6 : determining if the user's emotion reaching a neutral emotion through the physiological monitoring device;

step 7 : after the determination of the user's emotion is reaching the neutral emotion, performing a lighting program of the multispectral recipe corresponding to the target emotion, wherein the client terminal control a lamps group in the lighting field end to conducting a lighting process to the user for a set time according to the multispectral recipe corresponding to the target emotion;

step 8 : determining if the user's emotion reaching the target emotion through the physiological monitoring device; and

step 9 : stopping lighting when determination of the user's emotion is reaching the target emotion.

10. The emotion navigation method to claim 9, wherein the relay emotion of step 3 is selected the emotion that is complementary to the initial emotion of the user.

11. The emotion navigation method to claim 9, wherein the determination whether the user's emotion reaches the neutral emotion is to determine whether the user's emotion is neutral or near the origin of an emotion coordinate system.

12. The emotion navigation method to claim 9, wherein further includes a step 10 to obtain a personal physiological data of the user if the determination of the user's emotion is not reaching the neutral emotion.

13. The emotion navigation method to claim 12, wherein the personal physiological data is the physiological data of the user's health checkup.

14. The emotion navigation method to claim 12, wherein if the determination of the user's emotion is not reaching the neutral emotion, after adjusting the multispectral recipe corresponding to the relay emotion according to the user's personal physiological data, step 5 to step 6 are re-executed.

15. The emotion navigation method to claim 9, wherein the physiological monitoring device can an EEG, a mobile phone or a wearable device that can detect sympathetic and parasympathetic nerve signals.

16. The emotion navigation method to claim 9, wherein the multispectral recipe includes lighting parameters such as color temperature, illuminance, flicker frequency, and color rendering (Ra).

17. The emotion navigation method to claim 16, wherein the multispectral recipe is a formula formed by the surrounding system score (LSS).

$$LSS = t \times [a \times f_{eeg}(freq) + b \times f_{eeg}(Ra) + c \times f_{eeg}(CTI) + d \times f_{eeg}(I)]$$

$a = w_{freq}/w_{CTI}$
$b = w_{Ra}/w_{CTI}$
$c = w_{CTI}/w_{CTI} = 1$
$d = w_{I}/w_{CTI}$
$t$ = illumination time

18. A method for implementing emotion navigation by using an intelligent human centric lighting system, wherein the intelligent human centric lighting system is composed of a cloud, a lighting field end and a client terminal which are connected to each other through the Internet and an emotion coordinate information and users' personal biological data are stored in the cloud, and wherein the emotion navigation method including:

step 1 : confirming an initial emotion of a user through a physiological monitoring device or by using an International Affection Picture System (IAPS) to simulate for confirming the initial emotion of the user, and storing the initial emotion in the cloud;

step 2 : setting a target emotion according to a requirement of the user by the client terminal, and storing the target emotion in the cloud;

step 2 : obtaining the user's personal biological data stored in a memory module of the cloud by the client terminal;

step 4 : choosing a relay emotion to set an emotion navigation path and storing the relay emotion in the cloud, wherein the client terminal is connected the cloud through the Internet and the relay emotion is chosen from an emotion coordinate information in the cloud;

step 5 : editing a multispectral recipe according to the light recipe of the relay emotion and the light recipe of the target emotion and the user's personal biological data to edit the multispectral recipe corresponding to the relay emotion and the multispectral recipe corresponding to the target emotion;

step 6 : performing a lighting program of the multispectral recipe corresponding to the relay emotion, wherein the client terminal control a lamps group in the lighting field end to conducting a lighting process to the user for a set time according to the multispectral recipe corresponding to the relay emotion;

step 7 : determining if the user's emotion reaching a neutral emotion through the physiological monitoring device;

step 8 : after the determination of the user's emotion is reaching the neutral emotion, performing a lighting program of the multispectral recipe corresponding to the target emotion, wherein the client terminal control a lamps group in the lighting field end to conducting a lighting process to the user for a set time according to the multispectral recipe corresponding to the target emotion;

step 9: determining if the user's emotion reaching the target emotion through the physiological monitoring device; and

step 10 : stopping lighting when determination of the user's emotion is reaching the target emotion.

19. The emotion navigation method to claim 18, wherein the relay emotion of step 3 is selected the emotion that is complementary to the initial emotion of the user.

20. The emotion navigation method to claim 18, wherein the determination whether the user's emotion reaches the neutral emotion is to determine whether the user's emotion is neutral or near the origin of an emotion coordinate system.

21. The emotion navigation method to claim 18, wherein the personal physiological data is the physiological data of the user's health checkup.

22. The emotion navigation method to claim 18, wherein if the determinationt of the user's emotion is not reaching the neutral emotion, after adjusting the multispectral recipe corresponding to the relay emotion according to the user's personal physiological data, step 6 to step 7 are re-executed.

23. The emotion navigation method to claim 18, wherein the physiological monitoring device can an EEG, a mobile phone or a wearable device that can detect sympathetic and parasympathetic nerve signals.

24. The emotion navigation method to claim 18, wherein the multispectral recipe includes the lighting parameters such as color temperature, illuminance, flicker frequency, and color rendering (Ra).

25. The emotion navigation method to claim 18, wherein the multispectral recipe is a formula formed by the surrounding system score (LSS).

$$LSS = t \times [a \times f_{eeg}(freq) + b \times f_{eeg}(Ra) + c \times f_{eeg}(CTI) + d \times f_{eeg}(I)]$$

$a = w_{freq}/w_{CTI}$
$b = w_{Ra}/w_{CTI}$
$C = w_{CTI}/w_{CTI} = 1$
$d = w_I/w_{CTI}$

t = illumination time

100 — Start human centric lighting ← Change spectrum, light intensity, flashing rate, color

Recording hyperemia in brain by fMRI

Using EEG to record the changes of brain waves

Eye reaction was recorded by eye tracker

Using expression recognition technology to assist in judging emotion

110

130

150

170

# FIG.1a

The tester used known pictures to simulate various emotions in fMRI

1100

fMRI was used to record the hyperemia reaction of various emotions in the brain after being stimulated by pictures

1200

Provide spectra with different color temperature parameters by lighting

1300

fMRI was used to record the hyperemia reaction of various emotions in the brain after being stimulated by lighting

1400

Specific color temperatures which be filtered out through lighting can increase the brain's hyperemia response to specific emotions

1500

Establishing that specific color temperature has a hyperemic in the brain with response to a specific emotion in the brain

1600

# FIG.1b

FIG.1c

Load the enhanced spectrum
database to the EEG

2100

Taster wore EEG to watch
specific emotion evoked
pictures

2200

2300

2310

Expose and record
the brain wave
diagram of 3,000K
spectral stimulation
results

2320

Expose and record
the brain wave
diagram of 4,000K
spectral stimulation
results

2330

Expose and record
the brain wave
diagram of 5,700K
spectral stimulation
results

Transfer Learning Model

2400

Brainwave-Spectrum Classifier

2500

# FIG.2a

Testers wore EEG to watch specific emotion evoked pictures — 3100

Start human centric lighting — 3200

3400

Record the subject's EEG file — 3300

Load the Brainwave-Spectrum Classifier to the EEG

Using EEG to infer the state of hyperemia to the brain — 3500

Y

N

Stop human centric lighting — 3600

Continuously strengthen human centric lighting

3800

Establish the human centric lighting parameter data file to the tester — 3700

# FIG.2b

EP 4 703 000 A1

FIG.3

31

II · Activated: Red or Blue I

FIG.4a

electroencephalogram of nervous emotion

electroencephalogram of relaxed emotion

electroencephalogram of happiness emotion

Relative Strength = (BetaHigh +BetaLow) / (AlphaLow + AlphaHigh)

Relative Strength = (AlphaLow + AlphaHigh) / (BetaHigh +BetaLow)

Relative Strength = (AlphaLow + AlphaHigh + BetaHigh) / (BetaLow)

**FIG.4b**

electroencephalogram of
happiness emotion

5 4 3 2 1 0

AlphaLow AlphaHigh BetaLow BetaHigh

Relative Strength = 3.34

electroencephalogram of
nervous emotion

5 4 3 2 1 0

AlphaLow AlphaHigh BetaLow BetaHigh

Relative Strength = 3

FIG.4c

electroencephalogram of nervous emotion

Relative Strength = 2.94

electroencephalogram of relaxed emotion

Relative Strength = 1.66

electroencephalogram of happiness emotion

Relative Strength = 4

**FIG.4d**

**electroencephalogram of nervous emotion**

AlphaLow  AlphaHigh  BetaLow  BetaHigh

Relative Strength = 2.86

**electroencephalogram of relaxed emotion**

AlphaLow  AlphaHigh  BetaLow  BetaHigh

Relative Strength = 1.15

**electroencephalogram of happiness emotion**

AlphaLow  AlphaHigh  BetaLow  BetaHigh

Relative Strength = 10

**FIG.4e**

**FIG.4f**

FIG.4g

FIG.4h

3510 — Confirming initial emotion

3520 — Setting target emotion

Obtaining the user's personal physiological data

3530 — Setting emotion navigation path

3590

3540 — Editing spectral recipe according to emotion navigation path

3580 — Checking the physiological monitoring signal of the user

3550 — Execute lighting procedure of spectral recipe

3560 — Determining if the user's emotion reaching the target emotion

N

Y

3570 — Stopping the lighting procedure

FIG.5

4510 — Confirming initial emotion

4520 — Setting target emotion

4570 — Obtaining the user's personal physiological data

4530 — Setting emotion navigation path

4540 — Editing spectral recipe according to emotion navigation path

4550 — Execute the first stage lighting procedure

4560 — Determining if the user's emotion reaching the neutral

N

Y

4580 — 4590 — Execute the second stage lighting procedure

Determining if the user's emotion reaching the target emotion

N

Y

4610 — Stopping the lighting procedure

FIG.6

6510 — Confirming initial emotion

6520 — Setting target emotion

6530 — Obtaining the user's personal physiological data

6540 — Setting emotion navigation path

6550 — Editing spectral recipe according to emotion navigation path

6560 — Execute the first stage lighting procedure

6570 — Determining if the user's emotion reaching the neutral

N

Y

6580 — Execute the second stage lighting procedure

6590 — Determining if the user's emotion reaching the target emotion

N

Y

6610 — Stopping the lighting procedure

FIG.7

## INTERNATIONAL SEARCH REPORT

| | |
|---|---|
| International application No. | |
| | **PCT/CN2023/091059** |

| | |
|---|---|
| **A.    CLASSIFICATION OF SUBJECT MATTER** | |
| A61M21/02(2006.01)i | |

According to International Patent Classification (IPC) or to both national classification and IPC

| | |
|---|---|
| **B.    FIELDS SEARCHED** | |

Minimum documentation searched (classification system followed by classification symbols)

  IPC: A61M21, A61N5, A61B5

Documentation searched other than minimum documentation to the extent that such documents are included in the fields searched

Electronic data base consulted during the international search (name of data base and, where practicable, search terms used)

  CNTXT, CNABS, ENTXTC, ENTXT, VEN: 情绪, 导航, 调整, 调节, 调控, 控制, 训练, 引导, 中性情绪, 中间情绪, 中继情绪, 互补, 原点, 平静, 光, emotion+, mood, spirit, adjust+, regulat+, control+, train+, lead+, neutral, serene, complement+, zero, calm, led?, illumination

| | |
|---|---|
| **C.    DOCUMENTS CONSIDERED TO BE RELEVANT** | |

| Category* | Citation of document, with indication, where appropriate, of the relevant passages | Relevant to claim No. |
|---|---|---|
| A | CN 115379626 A (STRONGLED SMART LIGHTING (CAYMAN) CO., LTD. et al.) 22 November 2022 (2022-11-22)<br>    description, paragraphs 23-92, and figures 1-6 | 1-25 |
| A | CN 106492354 A (TONGJI UNIVERSITY) 15 March 2017 (2017-03-15)<br>    entire document | 1-25 |
| A | CN 109350827 A (FUDAN UNIVERSITY) 19 February 2019 (2019-02-19)<br>    entire document | 1-25 |
| A | CN 114191723 A (BEIJING AHLGUANG TECHNOLOGY CO., LTD.) 18 March 2022 (2022-03-18)<br>    entire document | 1-25 |
| A | WO 2023011941 A1 (KONINKLIJKE PHILIPS N.V.) 09 February 2023 (2023-02-09)<br>    entire document | 1-25 |

☐ Further documents are listed in the continuation of Box C.    ☑ See patent family annex.

| | |
|---|---|
| *    Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| | |
|---|---|
| Date of the actual completion of the international search<br><br>**12 December 2023** | Date of mailing of the international search report<br><br>**21 December 2023** |
| Name and mailing address of the ISA/CN<br><br>**China National Intellectual Property Administration (ISA/CN)**<br>**China No. 6, Xitucheng Road, Jimenqiao, Haidian District, Beijing 100088** | Authorized officer<br><br><br><br>Telephone No. |

Form PCT/ISA/210 (second sheet) (July 2022)

# EP 4 703 000 A1

**INTERNATIONAL SEARCH REPORT**
Information on patent family members

International application No.

**PCT/CN2023/091059**

| Patent document cited in search report | | | Publication date (day/month/year) | Patent family member(s) | | | Publication date (day/month/year) |
|---|---|---|---|---|---|---|---|
| CN | 115379626 | A | 22 November 2022 | US | 2022369995 | A1 | 24 November 2022 |
| | | | | TW | 202247000 | A | 01 December 2022 |
| | | | | TW | 202245688 | A | 01 December 2022 |
| | | | | US | 2022373985 | A1 | 24 November 2022 |
| | | | | US | 2022377867 | A1 | 24 November 2022 |
| | | | | TW | 202245687 | A | 01 December 2022 |
| | | | | TWI | 803151 | B | 21 May 2023 |
| | | | | TW | 202245692 | A | 01 December 2022 |
| | | | | TWI | 799087 | B | 11 April 2023 |
| | | | | TW | 202247706 | A | 01 December 2022 |
| | | | | WO | 2022242245 | A1 | 24 November 2022 |
| | | | | TW | 202247192 | A | 01 December 2022 |
| | | | | TWI | 799086 | B | 11 April 2023 |
| | | | | CN | 115363579 | A | 22 November 2022 |
| | | | | CN | 115373510 | A | 22 November 2022 |
| | | | | CN | 115376655 | A | 22 November 2022 |
| | | | | CN | 115379623 | A | 22 November 2022 |
| | | | | CN | 115444418 | A | 09 December 2022 |
| CN | 106492354 | A | 15 March 2017 | CN | 106492354 | B | 14 September 2018 |
| CN | 109350827 | A | 19 February 2019 | CN | 209917015 | U | 10 January 2020 |
| CN | 114191723 | A | 18 March 2022 | None | | | |
| WO | 2023011941 | A1 | 09 February 2023 | EP | 4132224 | A1 | 08 February 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- MRI BOLD Correlates of EEG Independent Components: Spatial Correspondence with the Default Mode Network. *Neuroscience Journal (Front. Hum. Neurosci)*, 27 November 2018